(19) Europäisches Patentamt
European Patent Office
Office européen des brevets

(11) EP 2 188 620 B1

(12) FASCICULE DE BREVET EUROPEEN

(45) Date de publication et mention de la délivrance du brevet:
**23.08.2017 Bulletin 2017/34**

(21) Numéro de dépôt: **08804305.4**

(22) Date de dépôt: **17.09.2008**

(51) Int Cl.:
***G01N 21/66*** (2006.01) ***G01N 21/76*** (2006.01)
***H01L 51/00*** (2006.01) ***C07F 17/00*** (2006.01)
***C07D 401/04*** (2006.01) ***C07D 401/14*** (2006.01)
***C07D 409/14*** (2006.01) ***H01L 51/50*** (2006.01)
***C07F 7/08*** (2006.01)

(86) Numéro de dépôt international:
**PCT/EP2008/062351**

(87) Numéro de publication internationale:
**WO 2009/037277 (26.03.2009 Gazette 2009/13)**

(54) **COMPOSES UTILES COMME LIGANDS ET NOTAMMENT COMME CHROMOPHORES ORGANIQUES DE COMPLEXATION DES LANTHANIDES ET LEURS APPLICATIONS**

VERBINDUNGEN ALS LIGANDEN UND IM BESONDEREN ALS ORGANISCHE CHROMOPHORE ZUR LANTHANIDENKOMPLEXBILDUNG UND IHRE AWENDUNGEN

COMPOUNDS USEFUL AS LIGANDS AND PARTICULARLY AS ORGANIC CHROMOPHORES FOR COMPLEXING LANTHANIDES AND APPLICATIONS THEREOF

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **17.09.2007 FR 0757640**

(43) Date de publication de la demande:
**26.05.2010 Bulletin 2010/21**

(73) Titulaire: **Commissariat à l'Énergie Atomique et aux Énergies Alternatives
75015 Paris (FR)**

(72) Inventeurs:
- **GIRAUD, Marion
75018 Paris (FR)**
- **DEMADRILLE, Renaud
F-38120 St Egreve (FR)**
- **MAZZANTI, Marinella
F-38950 St Martin Le Vinoux (FR)**
- **ANDREIADIS, Eugen
75018 Paris (FR)**

(74) Mandataire: **Ilgart, Jean-Christophe et al
BREVALEX
95, rue d'Amsterdam
75378 Paris Cedex 8 (FR)**

(56) Documents cités:
**EP-A- 1 746 094 WO-A-96/19459
WO-A-03/003008 WO-A-03/003009
WO-A-2005/118606 WO-A-2006/066968
US-A- 5 639 770**

- **ANDREWS, PHILIP C. ET AL: "Gelation of La(III) cations promoted by 5-(2-pyridyl)tetrazolate and water" CHEMICAL COMMUNICATIONS, vol. 31, 2006, pages 3317-3319, XP002486387**
- **FACCHETTI, ANTONIO ET AL: "Novel coordinating motifs for lanthanide(III) ions based on 5-(2-pyridyl)tetrazole and 5-(2-pyridyl-1-oxide)tetrazole. Potential new contrast agents" CHEMICAL COMMUNICATIONS, vol. 15, 2004, pages 1770-1771, XP002486388**
- **CHAN, KANNIE WAI-YAN ET AL: "Small molecular gadolinium(III) complexes as MRI contrast agents for diagnostic imaging" COORDINATION CHEMISTRY REVIEWS, vol. 251, no. 17-20, 2007, pages 2428-2451, XP002486389**
- **WU, LI-LAN ET AL: "Photophysical and electrochemical properties of heteroleptic tris-cyclometallated Ir(III) complexes" POLYHEDRON, vol. 26, no. 12, 2007, pages 2679-2685, XP002486390**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- WU, LI-LAN ET AL: "Photophysical and Electrochemical Properties of Blue Phosphorescent Iridium(III) Complexes" ORGANOMETALLICS, vol. 26, no. 8, 2007, pages 2017-2023, XP002486391
- LIN, PING ET AL: "Synthesis and structures of 5-(pyridyl)tetrazole complexes of Mn(II)" DALTON TRANSACTIONS, vol. 14, 2005, pages 2388-2394, XP002486392
- YEH, SHI-JAY ET AL: "New dopant and host materials for blue-light-emitting phosphorescent organic electroluminescent devices" ADVANCED MATERIALS, vol. 17, no. 3, 2005, pages 285-289, XP002486393
- DUATI, MARCO ET AL: "Enhancement of Luminescence Lifetimes of Mononuclear Ruthenium(II)-Terpyridine Complexes by Manipulation of the .sigma.-Donor Strength of Ligands" INORGANIC CHEMISTRY, vol. 42, no. 25, 2003, pages 8377-8384, XP002486394
- ANDREWS, PHILIP C. ET AL: "Synthesis and structural characterization of cationic, neutral and hydroxo-bridged lanthanoid (La, Gd, Ho, Yb, Y) bis 5-(2-pyridyl)tetrazolate complexes" POLYHEDRON, vol. 26, no. 18, 2007, pages 5406-5413, XP002486395
- WEIBEL N ET AL: "Engineering of Highly Luminescent Lanthanide Tags Suitable for Protein Labeling and Time-Resolved Luminescence Imaging", JOURNAL OF THE AMERICAN CHEMICAL SOCIETY, AMERICAN CHEMICAL SOCIETY, US, vol. 126, 1 January 2004 (2004-01-01), pages 4888-4896, XP002315969, ISSN: 0002-7863, DOI: 10.1021/JA031886K
- BUENZLI, JEAN-CLAUDE G.: "Benefiting from the Unique Properties of Lanthanide Ions", ACCOUNTS OF CHEMICAL RESEARCH CODEN: ACHRE4; ISSN: 0001-4842, vol. 39, no. 1, 2006, pages 53-61, DOI: 10.1021/AR0400894 10.1021/AR0400894

## Description

### DOMAINE TECHNIQUE

La présente invention se rapporte à l'utilisation de composés comprenant au moins un motif 2-(1-*H*-tétrazol-5-yl)-pyridine comme ligands des lanthanides et, plus spécialement, comme chromophores organiques de complexation de ces éléments.

Elle se rapporte également à des complexes de lanthanides utilisant ces composés comme chromophores organiques de complexation, ainsi qu'à de nouveaux composés à motif(s) 2-(1-*H*-tétrazol-5-yl)-pyridine, utiles comme ligands des lanthanides et, en particulier, comme chromophores organiques de complexation de ces éléments.

Les complexes selon l'invention sont susceptibles de trouver des applications dans de nombreux domaines. Ainsi, par exemple, ils peuvent être utilisés en photonique et en optoélectronique, notamment pour former des dispositifs d'émission de lumière comme des diodes électroluminescentes mais ils peuvent également être utilisés en biologie, par exemple pour la préparation de sondes luminescentes.

### ÉTAT DE LA TECHNIQUE ANTÉRIEURE

Selon les règles émises par l'International Union of Pure and Applied Chemistry (IUPAC), les lanthanides (Ln) correspondent à la série des éléments chimiques allant du Cérium (Z = 58) au Lutécium (Z = 71).

En général, les lanthanides forment leurs composés les plus stables lorsqu'ils sont à l'état d'oxydation +3 ; la structure électronique des ions $Ln^{III}$ est celle du Xénon pour le $La^{III}$ et correspond ensuite au remplissage des orbitales 4f jusqu'à [Xe] $4f^{14}$ pour le $Lu^{III}$.

Les lanthanides sont connus dans la littérature pour leurs propriétés luminescentes qui peuvent être mises à profit dans le cadre de nombreuses applications dans les domaines de la photonique, de l'optoélectronique et de la biologie (marquage et imageries optique et magnétique).

Actuellement, la plupart des études réalisées avec des complexes de lanthanides ont été orientées vers l'établissement de sondes luminescentes contenant des émetteurs de lumière visible à vie longue, notamment $Eu^{III}$ et $Tb^{III}$, ou des émetteurs du spectre du proche infrarouge tels que $Pr^{III}$, $Er^{III}$, $Yb^{III}$ ou $Nd^{III}$.

Les lanthanides sont particulièrement intéressants pour des applications en photonique et électronique car leurs propriétés d'émission sont uniques. En effet, ils présentent des bandes d'émissions très fines apportant une grande pureté à la couleur émise. Par ailleurs, la durée de vie des états excités est particulièrement longue et les rendements quantiques de luminescence sont élevés. En outre, les plages d'émission peuvent être adaptées et il est possible d'obtenir des complexes de lanthanides émettant dans la gamme de longueurs d'onde allant de l'ultraviolet (UV) au proche infrarouge (IR).

Les transitions 4f-4f étant interdites selon les règles de Laporte, le coefficient d'absorption des lanthanides est très faible et leur excitation directe demande l'utilisation de sources lasers à haute énergie.

Pour permettre une émission du métal avec des énergies plus faibles, il est nécessaire de sensibiliser le lanthanide en le complexant avec un chromophore organique adapté, qui est généralement un système conjugué à forte absorption dans l'UV-visible comme une dicétone, capable d'absorber les photons et de les transférer de manière efficace aux ions lanthanides. Les photons absorbés vont exciter la molécule et la faire passer dans un état singulet qui peut relaxer pour revenir à l'état fondamental ou encore passer dans un état triplet par conversion intersystème. Si l'état triplet du métal est inférieur, énergétiquement parlant, à celui du chromophore, il y a alors transfert d'énergie (par mécanisme de Forster ou de Dexter) de l'état triplet du chromophore vers l'état triplet du métal qui revient à l'état fondamental en émettant de la lumière.

Pour qu'un tel système fonctionne efficacement, c'est-à-dire qu'il présente un rendement quantique de luminescence intéressant, il est nécessaire que le chromophore absorbe les photons et les transfère efficacement au métal grâce à une bonne compatibilité de son état triplet avec celui du métal.

Un certain nombre d'architectures différentes ont été proposées à ce jour pour sensibiliser les ions lanthanides.

Dans la plupart des cas, les chromophores comprennent des motifs pyridine, bipyridine ou terpyridine capables de sensibiliser l'émission de lanthanides émettant dans le visible (Facchetti, A. et al., Chem. Comm.., 2004, 1770-1771). Ces motifs ont été fonctionnalisés avec des groupes acides carboxyliques dans l'optique de former des complexes stables avec les lanthanides (Latva et al., Journal of Luminescence 1997, 75, 149-169 **[1]**). Des rendements quantiques intéressants ont pu être obtenus pour les complexes ainsi formés. Cependant, leur stabilité reste faible.

Il a, par ailleurs, été montré, par Chatterton et al. (Angewandte Chemie, édition internationale en anglais 2005, 44, 7595-7598 [2**]**) et par Nonat et al. (Chemistry, a European Journal 2006, 12, 7133-7150 [3**]**), que l'introduction de trois groupements picolinate dans des architectures tripodales ou de quatre groupements picolinate dans une architecture tétrapodale est susceptible de conduire à des complexes de lanthanides stables et rigides, avec des rendements quantiques intéressants pour le terbium (45-60%) mais nettement plus faibles pour l'europium (4-7%).

Des dérivés de 2-hydroxyisophthalamide inclus dans des architectures tétrapodales se sont aussi révélés efficaces pour sensibiliser le terbium, conduisant à des rendements quantiques élevés pour ce lanthanide (50-60%) mais, en revanche, faibles pour l'europium (6%) (Petoud et al., Journal of the American Chemical Society 2003, 125, 13324-13325 **[4]**), tandis que des rendements quantiques satisfaisants (de l'ordre de 44%) ont été obtenus pour l'europium par l'introduction de 2-hydroxypyridones dans des architectures dipodales (Moore et al., Inorganic Chemistry 2007, 46, 5468-5470 **[5]**).

Par ailleurs, la littérature ne recense pas de rendements quantiques supérieurs à 0,27% pour le néodyme et encore cette valeur, publiée par Vögtle et ses collaborateurs (ChemPhysChem 2001, 2, 769-773 **[6]**) fait-elle figure d'exception, les rendements quantiques obtenus pour ce lanthanide étant typiquement de 0,10%.

Il serait donc souhaitable de disposer de composés qui, outre de former des complexes avec les lanthanides, soient capables de sensibiliser efficacement plusieurs d'entre eux et, en particulier, de sensibiliser à la fois l'europium, le terbium et le néodyme.

Les Inventeurs se sont donc fixé pour but de fournir de tels composés.

Ils se sont aussi fixé pour but que les synthèses de ces composés et des complexes de lanthanides soient faciles à mettre en oeuvre et ne fassent appel qu'à des réactions classiquement utilisées en chimie organique.

Ils se sont encore fixé pour but que ces composés permettent d'obtenir des complexes de lanthanides stables et, autant que possible, que cette stabilité existe aussi bien lorsque les complexes sont en solution que lorsqu'ils se trouvent à l'état solide.

Ils se sont en outre fixé pour but que les complexes de lanthanides ainsi obtenus présentent, lorsqu'ils sont à l'état solide, des longueurs d'onde d'excitation compatibles avec les substrats classiquement utilisés en optique tels que les substrats en verre ou en oxyde d'indium et d'étain (ITO).

Ils se sont enfin fixé pour but que lesdits composés permettent d'éliminer la présence de molécules d'eau dans la première sphère de coordination des lanthanides, au moins lorsque les complexes sont à l'état solide, afin d'éviter ou, à tout le moins, de réduire les phénomènes de désexcitation non radiatifs liés à l'oscillation des liaisons OH.

## EXPOSÉ DE L'INVENTION

Ces buts, et d'autres encore, sont atteints par l'invention qui propose, en premier lieu, l'utilisation d'un composé comprenant au moins un motif 2-(1-*H*-tétrazol-5-yl)-pyridine, c'est-à-dire un motif de formule (I) ci-après :

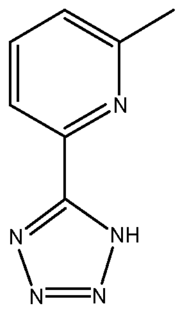

(I)

comme ligand d'un lanthanide et, plus spécialement, comme chromophore organique de complexation d'un lanthanide.

De préférence, le composé répond à la formule générale (II) ci-après :

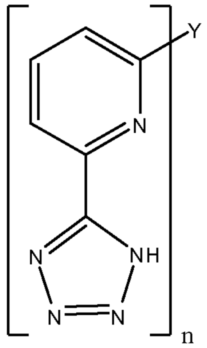

(II)

dans laquelle :

n est un nombre entier allant de 1 à 5 ; et

Y représente le reste d'une molécule organique qui est liée au(x) n motif(s) 2-(1-*H*-tétrazol-5-yl)-pyridine.

Selon une première modalité préférée de l'invention, Y représente un cycle aromatique qui comporte éventuellement un ou plusieurs hétéroatomes et/ou un ou plusieurs substituants.

Dans ce qui précède et ce qui suit, on entend par « *hétéroatome* », tout atome autre que de carbone ou d'hydrogène comme, par exemple, un atome d'oxygène, d'azote, de soufre, d'halogène, de phosphore, de bore ou encore de silicium, cet hétéroatome étant typiquement un atome d'azote, d'oxygène ou de soufre et, de préférence, d'azote lorsqu'il fait partie d'un cycle et ce, que ce cycle soit aromatique ou non.

Par ailleurs, bien que le terme *« substituant »* doive être pris, dans ce qui précède et ce qui suit, dans son acceptation la plus large, on préfère que les substituants soient des atomes d'halogène ou des groupes fonctionnels comprenant au moins un hétéroatome comme, par exemple, des groupes -COOR', -CHO, -OR', -SR', -SCOR', $-SO_2R'$, -NR'R", -CONR'R", $-C(Hal)_3$, $-OC(Hal)_3$, -C(O)Hal ou -CN dans lesquels R' et R" représentent un atome d'hydrogène ou un groupe alkyle, de préférence en $C_1$ à $C_3$, tandis que Hal représente un atome d'halogène, de préférence de fluor, de chlore ou de brome. Un substituant particulièrement préféré est le groupe acide carboxylique.

Le cycle aromatique susceptible d'être représenté par Y est, de préférence, un cycle pyridine, substitué ou non, auquel cas n est avantageusement égal à 1 ou 2 (ce qui signifie que ce cycle pyridine est lié à un ou deux motifs 2-(1-*H*-tétrazol-5-yl)-pyridine, ou bien un cycle tétrazole, substitué ou non, auquel cas n est avantageusement égal à 1 (ce qui signifie que ce cycle tétrazole est lié à un seul motif 2-(1-*H*-tétrazol-5-yl)-pyridine.

Lorsque Y est un cycle pyridine, on préfère, d'une part, que les atomes de carbone de ce cycle, qui sont situés en position méta de l'atome d'azote, ne soient pas substitués et, d'autre part, que le ou les motifs 2-(1-*H*-tétrazol-5-yl)-pyridine auxquels le cycle pyridine est lié soient situés en position ortho et/ou para dudit atome d'azote, ce type de configuration étant particulièrement favorable à la complexation du lanthanide par le composé de formule générale (II).

Ainsi, si le cycle pyridine est lié à un seul motif 2-(1-*H*-tétrazol-5-yl)-pyridine, alors ce motif est situé, de préférence, en position ortho ou para de l'atome d'azote de ce cycle tandis que, si le cycle pyridine est lié à deux motifs 2-(1-*H*-tétrazol-5-yl)-pyridine, alors ces deux motifs sont situés, de préférence, en position ortho de l'atome d'azote du cycle pyridine ou bien l'un de ces motifs est situé en position ortho tandis que l'autre est situé en position para dudit atome d'azote.

De manière particulièrement préférée, le composé répond à la formule (III) ci-après :

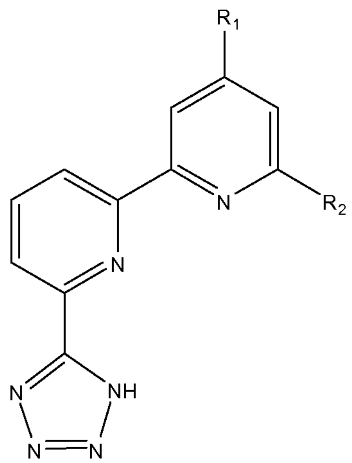

(III)

dans laquelle :

$R_1$ représente un atome d'hydrogène ou d'halogène ; un cycle aromatique à 5 ou 6 chaînons ou un enchaînement de plusieurs cycles aromatiques à 5 ou 6 chaînons, typiquement de 2 ou 3 cycles, liés les uns aux autres par une liaison covalente, ce cycle ou l'un au moins de ces cycles comportant éventuellement un ou plusieurs hétéroatomes, typiquement O, N ou S, et/ou un ou plusieurs substituants non cycliques ; ou encore un groupe hydrocarboné linéaire ou ramifié en $C_1$ à $C_{12}$ comportant éventuellement un ou plusieurs hétéroatomes ; et

$R_2$ représente un groupe acide carboxylique ; un cycle aromatique à 5 ou 6 chaînons, comportant éventuellement un ou plusieurs hétéroatomes, typiquement O, N ou S, et/ou un ou plusieurs substituants non cycliques ; ou encore un motif de formule (I) ci-avant.

Dans la formule (III) ci-avant, $R_1$ peut notamment être un groupe phényle ou thiophène, éventuellement substitué, en particulier par un ou plusieurs atomes d'halogène, comme le 4-bromophényle ou le 2-bromothiophène, ou encore un groupe diphényle, dithiophène, pyridin-2-yl-phényle, éventuellement substitué, en particulier par un ou plusieurs atomes d'halogène ou un ou plusieurs groupes alkyle en $C_1$ à $C_{12}$ ou silylalkyle en $C_3$ à $C_{12}$.

Lorsque Y est un cycle tétrazole, alors on préfère que ce cycle ne soit pas substitué.

Selon une autre modalité préférée de l'invention, Y représente un cycle non aromatique comportant éventuellement un ou plusieurs hétéroatomes, typiquement O, N ou S, et/ou un ou plusieurs substituants, ou un groupe de formule $-(CH_2)_p-Z$ où p est un nombre entier allant de 1 à 6 et Z représente un cycle aromatique ou non aromatique, comportant éventuellement un ou plusieurs hétéroatomes, typiquement O, N ou S, et/ou un ou plusieurs substituants.

Dans cette modalité, Y représente, de préférence, un groupe de formule $-(CH_2)_p-Z$ où p est égal à 1 ou 2 et Z est un cycle non aromatique de 5 à 9 chaînons. Concernant ce cycle non aromatique, il peut s'agir d'un cycle à 5 ou 6 chaînons tel que classiquement utilisé en chimie organique, par exemple cyclopentyle, cyclohexyle, cyclopentadiényle ou phényle, mais on préfère que ce cycle ait un nombre de chaînons plus élevé, par exemple de 9, et comporte de 2 à 5 hétéroatomes, avantageusement des atomes d'azote, auquel cas ce cycle est préférentiellement lié à autant de motifs 2-(1-*H*-tétrazol-5-yl)-pyridine qu'il comporte d'hétéroatomes.

Des exemples de tels cycles sont les cycles triazacyclononane et tétraazacyclononane.

Conformément à l'invention, Y peut aussi représenter un groupe dénué de cycle. Ainsi notamment, Y peut représenter un groupe acide carboxylique ou un groupe hydrocarboné linéaire ou ramifié en $C_1$ à $C_{12}$, ce groupe comportant éventuellement un ou plusieurs hétéroatomes et/ou un ou plusieurs substituants.

Des composés de formule générale (II) particulièrement préférés répondent aux formules particulières (II-a) à (II-k) ci-après :

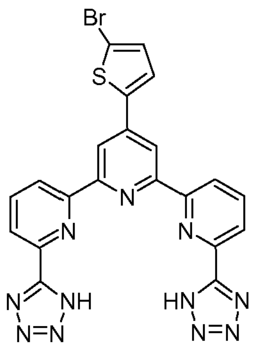

(II-a)

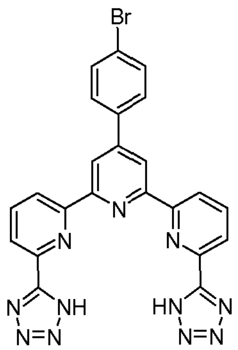

(II-b)

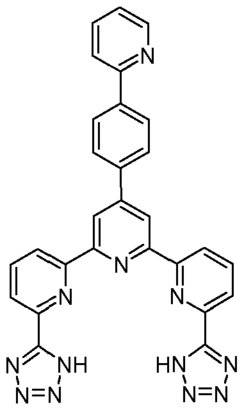

(II-c)

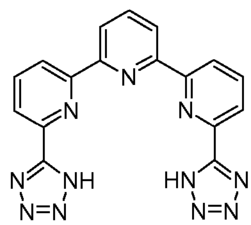

(II-d)

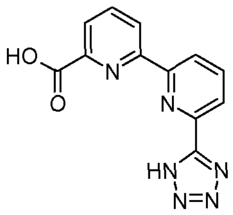

(II-e)

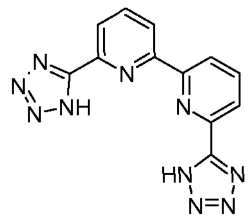

(II-f)

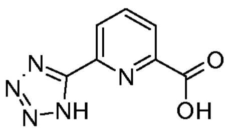

(II-g)

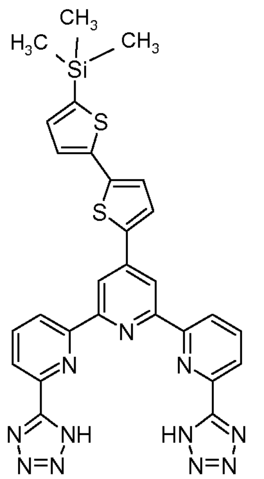

(II-h)

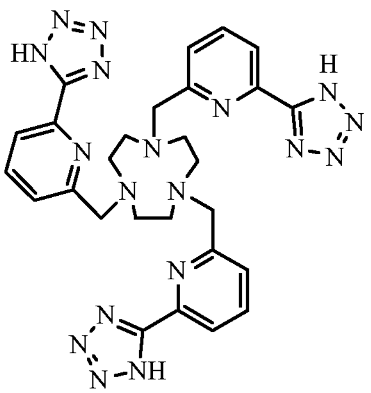

(II-i)

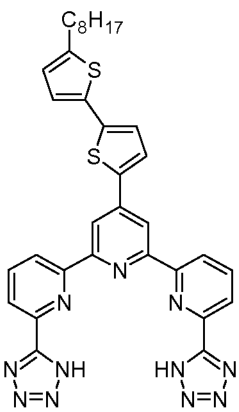

(II-j)

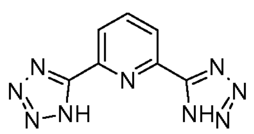

(II-k)

Selon encore une autre modalité préférée de l'invention, le lanthanide est choisi parmi l'europium, le terbium

et le néodyme.

L'invention a également pour objet un complexe d'un composé tel que précédemment défini et d'un lanthanide, dans lequel ledit composé joue le rôle de chromophore organique de complexation.

Un tel complexe peut être représenté par la formule (IV) ci-après :

$$[Ln(COM)_m] \qquad (IV)$$

dans laquelle :

Ln représente le lanthanide ;
COM représente le chromophore organique de complexation ; et
m est un nombre entier correspondant au nombre de molécules du chromophore organique de complexation qui sont liées au lanthanide, lequel nombre dépend du nombre de sites de coordination présentés par le lanthanide.

Généralement, le lanthanide est à l'état d'oxydation +3, auquel cas il présente 8 à 12 sites de coordination et, typiquement, 9.

Selon une modalité préférée de l'invention, le complexe comprend au total 3, 4 ou 5 motifs 2-(1-*H*-tétrazol-5-yl)-pyridine, sachant qu'un motif de ce type permet de former deux liaisons de coordination avec le lanthanide. Ainsi, par exemple, m est égal à 2 et le complexe comprend au total 4 motifs 2-(1-*H*-tétrazol-5-yl)-pyridine ; m est égal à 1 et le complexe comprend au total 3 motifs 2-(1-*H*-tétrazol-5-yl)-pyridine ou bien m est égal à 4 et le complexe comprend au total 4 motifs 2-(1-*H*-tétrazol-5-yl)-pyridine.

Selon une autre modalité préférée de l'invention, le complexe comprend, comme chromophore organique de complexation, un composé qui répond soit à la formule (III) représentée ci-avant, dans laquelle $R_1$ et $R_2$ ont la même signification que précédemment, soit à la formule générale (II) représentée ci-avant et dans laquelle Y représente un groupe de formule $-(CH_2)_p$-Z où p est égal à 1 ou 2 et Z est un cycle non aromatique de 5 à 9 chaînons.

Plus spécifiquement, le complexe comprend un composé qui répond à l'une quelconque des formules particulières (II-a), (II-b), (II-c), (II-d), (II-h), (II-i) et (II-j) représentées ci-avant.

Par ailleurs, le lanthanide est préférentiellement l'europium, le terbium ou le néodyme.

Parmi les composés susceptibles d'être utilisés comme ligands de lanthanides conformément à l'invention, certains sont connus en tant que composés chimiques, tandis que d'autres semblent ne jamais avoir été décrits dans la littérature.

L'invention a donc encore pour objet un composé qui répond à la formule générale (II) représentée ci-avant et dans laquelle :

n est un nombre entier allant de 1 à 5 ;
Y représente un cycle aromatique ou non aromatique, qui est lié au (x) n motif(s) 2-(1-*H*-tétrazol-5-yl)-pyridine et qui comporte éventuellement un ou plusieurs hétéroatomes, typiquement O, N ou S, et/ou un ou plusieurs substituants, ou un groupe de formule - $(CH_2)_p$-Z où p est un nombre entier allant de 1 à 6 et Z représente un cycle aromatique ou non aromatique, comportant éventuellement un ou plusieurs hétéroatomes, typiquement O, N ou S, et/ou un ou plusieurs substituants, ce groupe étant lié au(x) n motif(s) 2-(1-*H*-tétrazol-5-yl)-pyridine.

Dans ce composé, les particularités du cycle (aromatique ou non aromatique) et du groupe de formule $-(CH_2)_p$-Z susceptible d'être représenté par Y sont telles que définies précédemment. Il en est de même pour n.

Conformément à l'invention, le composé répond, de préférence, à l'une quelconque des formules particulières (II-a), (II-b), (II-c), (II-d), (II-e), (II-f), (II-h), (II-i) et (II-j) représentées ci-avant.

L'invention sera mieux comprise à la lumière du complément de description, qui se réfère à des exemples de réalisation de ligands et de complexes de lanthanides conformes à selon l'invention et de démonstration de leurs propriétés.

Bien entendu, ces exemples ne sont donnés qu'à titre d'illustrations de l'invention et n'en constituent en aucun cas une limitation.

**BRÈVE DESCRIPTION DES DESSINS**

La figure 1 représente la structure d'un premier complexe d'europium selon l'invention telle que résolue par diffraction des rayons X.
La figure 2 représente la structure d'un complexe de terbium selon l'invention telle que résolue par diffraction des

rayons X.

La figure 3 représente la structure d'un complexe de néodyme selon l'invention telle que résolue par diffraction des rayons X.

La figure 4 représente la structure d'un deuxième complexe d'europium selon l'invention telle que résolue par diffraction aux rayons X.

La figure 5 représente la structure d'un troisième complexe d'europium selon l'invention telle que résolue par diffraction aux rayons X.

La figure 6 représente la structure d'un quatrième complexe d'europium selon l'invention telle que résolue par diffraction aux rayons X.

La figure 7 montre les spectres d'excitation électronique d'un complexe d'europium selon l'invention (courbe A) et d'un complexe d'europium de l'art antérieur (courbe B) ainsi que les spectres d'absorption d'un substrat en verre (courbe S1) et d'un substrat en ITO (courbe S2).

## EXPOSÉ DÉTAILLÉ DE MODES DE RÉALISATION PARTICULIERS

Dans les exemples qui suivent, les solvants et les réactifs ont été obtenus auprès des sociétés Aldrich, Fluka et Acros et ont été utilisés sans purification supplémentaire.

Les triflates de lanthanides ont été fournis par la société Aldrich et leur teneur en métal a été titrée avant utilisation, en présence d'EDTA et de xylène orange.

Les spectres RMN $^1H$ et RMN $^{13}C$ ont été enregistrés sur un spectromètre Brüker AM-200 ou Varian U-400 et traités avec le logiciel MestRe-C, version 4.9. La signature complète des protons des complexes a été réalisée au moyen d'expériences NOESY (**N**uclear **O**verhauser **E**ffect **S**pectroscop**Y**).

Les analyses élémentaires ont été réalisées par le Service Central d'Analyses (Vernaison, France).

Les spectres d'absorption ont été enregistrés sur un spectrophotomètre UV-visible Varian Cary 50. Les mesures de luminescence basse résolution en solution (temps de vie, états triplets et rendements quantiques) ont été mesurés sur un spectromètre Perkin-Elmer LS-50B à 298K.

Pour les mesures des spectres et des états triplets à l'état solide, le spectromètre a été adapté à l'aide d'un système de refroidissement à l'azote liquide. Les mesures des états singulets ont été effectuées dans le méthanol à température ambiante tandis que les mesures des états triplets ont été réalisés à l'état solide à 77K avec un délai de 0,2 ms. Les mesures à l'état solide ont également été réalisées sur un spectromètre Spex Horiba Jobin Yvon Fluorolog® FL 3-22 avec un double monochromateur et un photomultiplicateur Hamamatsu Photonics R-928P.

Pour les mesures dans le proche infrarouge, le spectromètre a été adapté avec un canal de mesure équipé d'un monochromateur FL-1004 et la puissance de l'irradiation a été mesurée au moyen de deux détecteurs Jobin Yvon InGaAs : un détecteur DSS-IGA020L (domaine : 800-1600 nm) refroidi à 77K, et un détecteur DSS-IGA020A (domaine : 800-1700 nm) travaillant à température ambiante, inséré dans un habitacle LN2 comprenant un miroir elliptique (90°) et couplé à un système d'acquisition Jobin Yvon SpectrAcq2.

Tous les spectres ont été corrigés.

Les temps de vie de luminescence ont été mesurés en enregistrant la décroissance au maximum du spectre et le signal obtenus est modélisé par une fonction monoexponentielle sous Origin® 7.5. Les valeurs rapportées correspondent à la moyenne de 3 mesures indépendantes. Les solutions pour les mesures de rendements quantiques ont une absorbance de 0,2 dans une cellule en quartz Suprasil® de 2 mm d'épaisseur. Les cellules pour la luminescence ont été préparées par dissolution des complexes isolés dans du méthanol de qualité spectroscopique.

Les rendements quantiques Φ ont été calculés avec l'équation :

$$\Phi_x/\Phi_r = A_r(\lambda)\,n_x^2 D_x \;/\; A_S(\lambda)\,n_r^2 D_r$$

dans laquelle :

- x est relié à l'échantillon,
- r est relié à la référence,
- A est l'absorbance à la longueur d'onde d'excitation,
- n est l'indice de réfraction, et
- D est l'intensité émise intégrée sur l'ensemble du spectre.

Les complexes tris(dipicolinate) $[Eu(dpa)_3^{3-}]$ (Φ = 24%, 7,5. $10^{-5}$ M dans du tampon Tris 0,1 M) et $[Tb(dpa)_3^{3-}]$ (Φ = 22%, 6,5. $10^{-5}$ M dans du tampon Tris 0,1 M) ont été utilisés comme références pour déterminer les rendements

quantiques de l'europium et du terbium respectivement (Chauvin et al., Spectroscopy Letters 2004, 37, 517-532 **[7].** L'erreur de ce type de mesures est habituellement considérée de 15%.

Les rendements quantiques à l'état solide ont été déterminés avec le spectromètre Fluorolog FL 3-22 avec une sphère d'intégration sur mesure de chez Oriel et la procédure est décrite par de Mello et al. (Advanced Materials 1997, 9, 230-232 [8**]**). Les spectres ont été corrigés en fonction des instruments avec une méthode absolue avec une sphère d'intégration.

Concernant les études cristallographiques aux rayons X, toutes les données de diffraction ont été collectées avec un diffractomètre BRUKER Smart CCD avec un détecteur 3 cercles $2\theta_{max}$=59, 38° (radiation Mo $K\alpha$, monochromateur graphite, $\lambda$ = 0,71073 Å). Pour réduire la perte de solvant, les cristaux ont été recouverts d'une huile hydrocarbonée légère et transférés sous azote dans le diffractomètre. Les intensités avec I > 10$\sigma$ (I) détectées dans les réseaux en utilisant le programme Brücker Sadabs ont été utilisées pour affiner les valeurs des paramètres de maille. Les groupes d'espace ont été déterminés de manière systématique et confirmés par résolution des structures résolues par les méthodes directes en utilisant le programme SHELXTL 6.10.

**<u>EXEMPLE 1</u> : Complexes du 2,2'-6',2"-terpyridine-6,6"-ditétrazole et de divers lanthanides (europium, terbium et néodyme)**

**1.1. <u>Synthèse du 2,2'-6',2"-terpyridine-6,6"-ditétrazole</u>**

Le composé titre, noté ci-après **TPDTZ,** qui correspond au composé de formule particulière (II-d) représentée ci-avant, est synthétisé en partant du [2,2'-6',2"-terpyridine] -6, 6"-dicarbonitrile, noté ci-après composé **1,** selon le schéma réactionnel suivant :

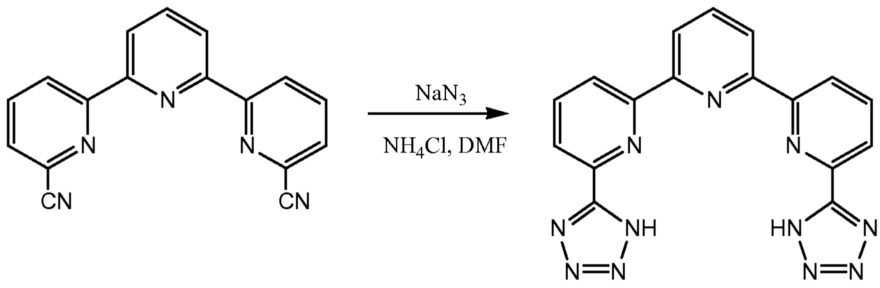


**1** **TPDTZ**

** Synthèse du composé **1***

Le composé **1** est synthétisé comme décrit par Mukkala et al. dans Helvetica Chimica Acta 1992, 75, 1621-1632 **[9].**

** Synthèse du TPDTZ*

Un mélange de 1,133 g (4,51 mmol) de composé **1,** de 1,300 g (20 mmol) d'azide de sodium ($NaN_3$) et de 1,07 g (20 mmol) de chlorure d'ammonium ($NH_4Cl$) dans 36 ml de diméthylformamide (DMF) anhydre est mis à réagir sous argon à 125-130°C pendant 16 heures. Après refroidissement, les sels inorganiques sont filtrés et le solvant est éliminé sous pression réduite. Le résidu est repris dans de l'acide chlorhydrique dilué (0,1 M, ~16 ml, pH ~ 2-3), agité pendant 1 heure et récupéré par filtration. Le produit est rincé à l'eau froide, séché sur le filtre puis sous vide.

On obtient ainsi 1,505 g de **TPDTZ** (Rdt : 90%).

**RMN $^1$H (200 MHz, DMSO) $\delta$ ppm :** 9.29 (d, J = 7.83 Hz, 2H), 9.24 (dd, J = 6.65, 2.36 Hz, 2H), 8.72-8.63 (m, 5H)
**RMN $^{13}$C (50 MHz, DMSO) $\delta$ ppm :** 155.70, 155.29, 153.94, 144.42, 139.20, 138.60, 122.62, 122.04, 121.97.

1.2. **<u>Synthèse d'un complexe de TPDTZ et d'europium</u>**

Une suspension de 95,16 mg (0,258 mmol) de **TPDTZ** dans 6 mL de méthanol est traitée avec 72 $\mu$L (52,14 mg) de triéthylamine (TEA) et soumise aux ultrasons. Puis, 77,19 mg de triflate d'europium dans 2 mL de méthanol sont ajoutés. La solution, qui vire légèrement au jaune, est laissée à température ambiante pour cristallisation. Le produit

résultant est filtré, lavé avec un faible volume de méthanol, puis d'éther, et séché à l'air.

On obtient ainsi 66,1 mg du complexe dont la structure, telle que résolue par diffraction des rayons X, est montrée sur la figure 1, sous la forme de cristaux blancs jaunâtres (Rdt : 51,8%).

**RMN $^1$H (400 MHz, MeOD)** $\delta$ **ppm :** 15.51 (d, J = 8.11 Hz, 2H), 12.20 (t, J = 7.83, 7.83 Hz, 2H), 5.33 (d, J = 7.80 Hz, 2H), 3.10 (q, J = 7.32, 7.30, 7.30 Hz, 3H), 1.21 (t, J = 7.31, 7.31 Hz, 4.5H) 0.72 (t, J = 7.52, 7.52 Hz, 1H), -0.50 (d, J = 7.59 Hz, 2H)

**Analyse élémentaire :** calculé (trouvé) pour $[Eu(TPDTZ)_2]NHEt_3 \cdot 1{,}25MeOH \cdot 1{,}5H_2O$ :

C : 46, 92% (46, 96%)
H : 4,01% (3, 86%)
N : 30,51% (30,21%)

**Diffraction des rayons X :**
Système cristallin monoclinique
Groupe d'espace C2/c
Paramètres de maille :

a = 16,764 (4) Å $\alpha$ = 90°
b = 18,295 (5) Å $\beta$ = 93, 207 (5) °
c = 14,430 (4) Å $\gamma$ = 90°

indices R (toutes données) R1 = 0,0360

### 1.3. Synthèse d'un complexe de TPDTZ et de terbium

Une suspension de 82,66 mg (0,224 mmol) de **TPDTZ** dans 5 mL de méthanol est traitée avec 62,4 $\mu$L de TEA et soumise aux ultrasons. Puis, 67,8 mg de triflate de terbium dans 3 mL de méthanol sont ajoutés. La solution opalescente résultante est filtrée et le filtrat est laissé à température ambiante pour cristallisation. Les cristaux ainsi formés sont récupérés par filtration, lavés avec un faible volume de méthanol, puis d'éther, et séchés à l'air.

Par évaporation partielle de la liqueur mère, on obtient une deuxième série de cristaux.

On obtient au total 53,6 mg du complexe dont la structure, telle que résolue par diffraction des rayons X, est montrée sur la figure 2, sous la forme de cristaux blancs (Rdt : 48%)

**RMN $^1$H (400 MHz, MeOD)** $\delta$ **ppm** : 95.43 (2H), 91.87 (1H), 4.22 (q, J = 7.00, 7.00, 7.00 Hz, 3H), 2.20 (t, J = 7.06, 7.06 Hz, 4.5H), -4.58 (2H), -67.59 (2H), -140.08 (2H). En raison de l'effet paramagnétique très puissant du terbium, les multiplicités des signaux n'ont pas pu être déterminées.

**Analyse élémentaire :** calculé (trouvé) pour $[Tb(TPDTZ)_2]NHEt_3 \cdot 4{,}5H_2O \cdot 0{,}15NHEt_3Otf$ :

C : 44, 4% (44,22%)
H : 4, 11% (3,63%)
N : 29, 09% (29, 08%)

**Diffraction des rayons X :**
Système cristallin monoclinique
Groupe d'espace C2/c
Paramètres de maille :

a = 16,719 (4) Å $\alpha$ = 90°
b = 18,270 (4) Å $\beta$ = 93, 225 (6) °
c = 14,453 (3) Å $\gamma$ = 90°

indices R (toutes données) R1 = 0,0488

### 1.4. Synthèse d'un complexe de TPDTZ et de néodyme

Une suspension de 94,49 mg (0,256 mmol) de **TPDTZ** dans 5 mL de méthanol est traitée avec 71,3 μL de TEA et soumise aux ultrasons. Puis, 75,7 mg de triflate de néodyme dans 1 mL de méthanol sont ajoutés. La solution opalescente résultante est filtrée et le filtrat est laissé à température ambiante pour cristallisation. Les cristaux ainsi formés sont récupérés par filtration, lavés avec un faible volume de méthanol, puis d'éther, et séchés à l'air.

On obtient ainsi 51,9 mg du complexe dont la structure, telle que résolue par diffraction des rayons X, est montrée sur la figure 3, sous la forme de cristaux blancs (Rdt : 41,3%).

**RMN $^1$H (400 MHz, MeOD) δ ppm :** 16.18 (d, J = 6.3 Hz, 2H), 14.99 (t large, 1H), 11.37 (d, J = 7.07 Hz, 2H), 5.84 (t large, 2H), 3.11 (q, J = 7.30, 7.30, 7.28 Hz, 3H), 2.06 (d, J = 6.45 Hz, 2H), 1.22 (t, J = 7.30, 7.30 Hz, 4.5H)

**Diffraction des rayons X :**

Système cristallin monoclinique

Groupe d'espace C2/c

Paramètres de maille :

a = 16, 901 (4) Å    α = 90°

b = 18, 198 (5) Å    β = 93, 225 (6) °

c = 14, 395 (4) Å    γ = 90°

indices R (toutes données) R1 = 0,0623

**<u>EXEMPLE 2</u> : Complexe de 2,2'-6',2"-terpyridine-4'-(p-bromophényl)-6,6"-ditétrazole et d'europium**

### 2.1. Synthèse du 2,2'-6',2"-terpyridine-4'-(p-bromophényl)-6,6"-ditétrazole

Le composé titre, noté ci-après **TPDTZPB,** qui correspond au composé de formule particulière (II-b) représentée ci-avant, est synthétisé en partant du [2,2'-6',2"-terpyridine] -4'- (p-bromophényl) -6, 6"-dicarbonitrile, noté ci-après composé **2,** selon le schéma réactionnel suivant :

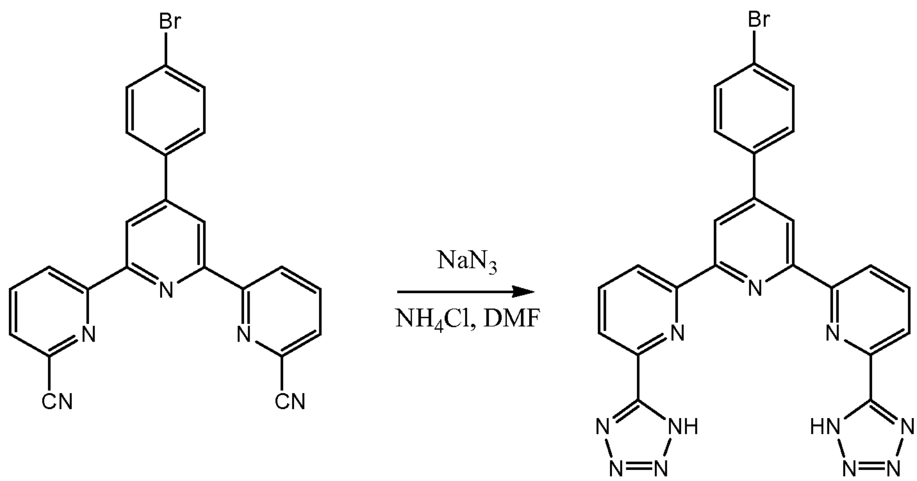


**2** **TPDTZPB**

** Synthèse du composé **2***

Le composé **2** est synthétisé comme décrit par Hovinen et al., Organic Letters 2001, 3, 2473-2476 **[10].**

** Synthèse du **TPDTZPB***

Un mélange de 435 mg (0,99 mmol) de composé **2,** de 325 mg (5 mmol) de $NaN_3$ et 267 mg (5 mmol) de $NH_4Cl$ dans 20 mL de DMF anhydre est mis à réagir sous argon pendant 20 heures à 130°C. Après refroidissement, on récupère par filtration un précipité qui est traité avec de l'acide chlorhydrique dilué, agité pendant 1 heure et réfrigéré.

Après filtration, lavage à l'eau froide et séchage sous vide, on obtient 454 mg de **TPDTZPB** sous la forme d'une poudre jaune clair (Rdt : 87%).

Le rendement peut être amélioré en soumettant le résidu, après évaporation du DMF, à un traitement à l'HCl dilué similaire à celui appliqué au précipité.

**RMN $^1$H (200 MHz, DMSO)** $\delta$ **ppm :** 9.04 (s, 2H), 8.79 (dd, J = 7.18, 1.53 Hz, 2H), 8.30-8.17 (m, 4H), 8.05 (d, J = 8.54 Hz, 2H), 7.86 (d, J = 8.40 Hz, 2H), 3.86 (large)

**RMN $^{13}$C (50 MHz, DMSO)** $\delta$ **ppm :** 156.62, 154.89, 148.60, 145.56, 138.96, 136.42, 132.09, 129.32, 123.12, 122.52, 121.84, 118.73.

**2.2. <u>Synthèse du complexe de TPDTZPB et d'europium</u>**

On ajoute, à une suspension de 90,96 mg (0,1735 mmol) de **TPDTZPB** dans 5 mL de méthanol, 48,36 $\mu$L de TEA, puis 51,97 mg de triflate d'europium, ce qui conduit à une solution opalescente. Par chauffage, l'opalescence disparaît et un fin précipité cristallin commence à se former. La solution est laissée à température ambiante toute une nuit pour cristallisation. Le produit résultant est filtré et lavé avec un faible volume d'éthanol et séché à l'air.

On obtient ainsi 87,8 mg du complexe dont la structure, telle que résolue par diffraction des rayons X, est montrée sur la figure 4, sous la forme de cristaux blancs jaunâtres (Rdt : 77,9%).

**RMN $^1$H (400 MHz, MeOD)** $\delta$ **ppm :** 15.70 (d, J = 7.98 Hz, 2H), 12.27 (t, J = 7.89, 7.89 Hz, 2H), 6.06 (d, J = 8.94 Hz, 2H), 5.40 (d, J = 7.79 Hz, 2H), 4.08 (d, J = 9.03 Hz, 2H), 3.17 (q, J = 7.32, 7.32, 7.31 Hz, 3H), 1.28 (t, J = 7.31, 7.31 Hz, 4.5H), -0.30 (s, 2H)

**Analyse élémentaire** : calculé (trouvé) pour $[Eu(TPDTZPB)_2]NHEt_3 \cdot 1,25MeOH \cdot 2,8H_2O$ :

C : 46, 04% (46, 03%)
H : 3,67% (3,26%)
N : 23,19% (23,15%)

**Diffraction des rayons X :**
Système cristallin monoclinique
Groupe d'espace C2/c
Paramètres de maille :

| | |
|---|---|
| a = 22,628 (10) Å | $\alpha$ = 90° |
| b = 19,032 (8) Å | $\beta$ = 107, 356 (7) ° |
| c = 14, 977 (7) Å | $\gamma$ = 90° |

indices R (toutes données) R1 = 0,1025

**<u>EXEMPLE 3</u> : Complexes de 4'-(5-bromo-2-thiényl)-2,2'-6'-2"-terpyridine-6,6"-ditétrazole et de divers lanthanides (europium et néodyme)**

3.1. **<u>Synthèse du 4'-(5-bromo-2-thiényl)-2,2'-6'-2"-terpyridine-6,6"-ditétrazole</u>**

Le composé titre, noté ci-après **TPDTZTB,** qui correspond au composé de formule particulière (II-a) représentée ci-avant, est synthétisé en partant de la 4'-(5-bromo-2-thiényl)-2,2'-6',2"-terpyridine, notée ci-après composé **3,** selon le schéma réactionnel suivant :

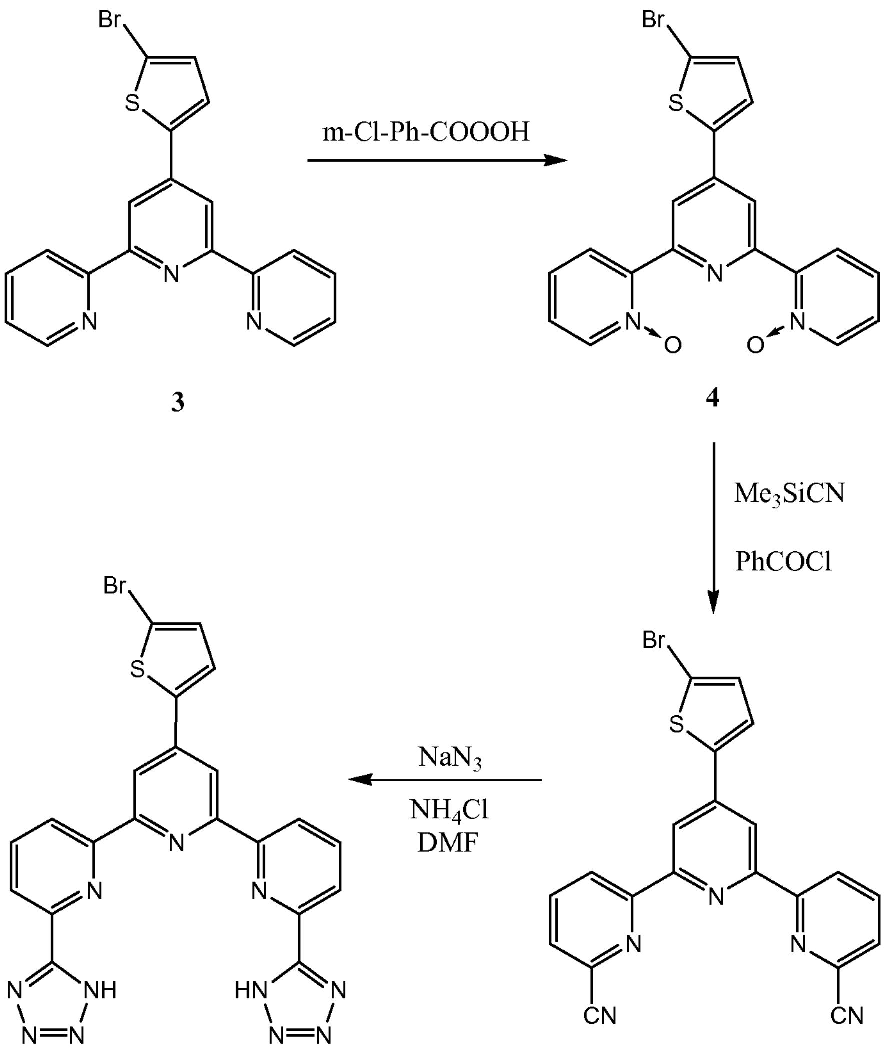


3

4

TPDTZTB

5

** Synthèse du composé **3***

Le composé **3** est synthétisé comme décrit par Ferraudi, Moya et ses collaborateurs dans López et al., Inorganica Chimica Acta 2004, 357, 3525-3531 **[11].**

** Synthèse du 4'- (5-bromo-2-thiényl) -2,2'-6'-2"-terpyridine-1,1"-dioxyde ou composé **4***

Une suspension de 5,62 g (22,8 mmol) d'acide 3-chloroperbenzoïque à 70% dans du dichlorométhane ($CH_2Cl_2$) est ajoutée lentement à une solution contenant 2,40 g (6,09 mmol) de composé **3** dans 50 mL de $CH_2Cl_2$. Le mélange est agité pendant 18 heures à température ambiante. Puis, il est lavé avec une solution de carbonate de sodium à 10%, séché sur $Na_2SO_4$ et concentré. Une purification par chromatographie sur une alumine avec de l'acétate d'éthyle et de l'éthanol permet d'obtenir 1,55 g d'une poudre blanche (Rdt : 60%).

> **RMN $^1$H (200 MHz, $CDCl_3$) $\delta$ ppm** : 9.14 (s, 2H), 8.35 (dd, J = 6.06, 1.32 Hz, 2H), 8.23 (dd, J = 7.75, 2.37 Hz, 2H), 7.44 (d, J = 3.40 Hz, 1H), 7.36 (td, J = 7.70, 1.36 Hz, 2H), 7.32 (td, J = 7.50, 2.30 Hz, 2H), 7.10 (d, J = 3.94, 1H)

** Synthèse du 4'- (5-bromo-2-thiényl) -2,2'-6'-2"-terpyridine-6,6"-dicarbonitrile ou composé **5***

Une solution de 3 g (7,04 mmol) de composé **4** et de 7 g (70 mmol) de cyanotriméthylsilane ($Me_3SiCN$) dans 100 mL de $CH_2Cl_2$ est agité à température ambiante pendant 20 minutes. Puis, 3,95 g (28 mmol) de chlorure de benzoyle sont ajoutés goutte à goutte et la solution résultante est agitée à température ambiante pendant 20 heures. Par filtration,

on obtient un précipité qui est lavé à l'eau, au méthanol et séché.

On obtient ainsi 2,00 g de composé **5** sous la forme d'un solide blanc jaunâtre (Rdt : 64%).

**RMN $^1$H (200 MHz, DMSO, 80°C) δ ppm :** 8.80 (dd, J = 8.11, 0.95 Hz, 2H), 8.59 (s, 2H), 8.01 (t, J = 7.87, 7.87 Hz, 2H), 7.76 (dd, J = 7.60, 0.96 Hz, 2H), 7.55 (d, J = 3.95 Hz, 1H), 7.16 (d, J = 3.94 Hz, 1H)

** Synthèse du **TPDTZTB***

Un mélange de 633 mg (1,425 mmol) de composé **5,** de 463 mg (7,12 mmol) de $NaN_3$ et de 381 mg (7,12 mmol) de $NH_4Cl$ dans 15 mL de DMF anhydre est agité sous argon à 140°C pendant 20 heures. Après refroidissement, on récupère par filtration un précipité que l'on traite avec de l'acide chlorhydrique dilué, sous agitation pendant 1 heure. La phase organique est évaporée et le résidu est repris dans de l'HCl dilué, soumis aux ultrasons et agité pendant 1 heure. Les suspensions acides sont réunies, filtrées, lavées à l'eau froide et séchées sous vide.

On obtient ainsi 630 mg de **TPDTZTB** sous la forme d'une poudre jaune brun (Rdt : 90%).

**RMN $^1$H (200 MHz, DMSO) δ ppm** : 8.86 (s, 2H), 8.77 (d, J = 7.18 Hz, 2H), 8.30 (d, J = 6.91 Hz, 2H), 8.22 (t, J = 7.60 Hz, 7.60 Hz, 2H), 7.94 (d, J = 3.88 Hz, 1H), 7.47 (d, J = 3.80 Hz, 1H)
**RMN $^{13}$C (50 MHz, DMSO) δ ppm** : 154.84, 154.46, 143.02, 142.38, 142.02, 139.36, 131.91, 127.98, 122.91, 122.83, 116.90, 114.46

**3.2. <u>Synthèse d'un complexe de TPDTZTB et d'europium</u>**

98,03 mg (0,185 mmol) de **TPDTZTB** en suspension dans 5 mL de méthanol sont traités avec 51,5 μL de TEA et soumis aux ultrasons. Puis, 55,4 mg de triflate d'europium dans 2 mL de méthanol sont ajoutés. Un précipité jaune orangé se forme instantanément qui est complètement redissous en 3 minutes environ par chauffage. La solution résultante est filtrée et laissée toute une nuit à température ambiante. On obtient ainsi de fins cristaux que l'on récupère par filtration, que l'on lave avec un faible volume d'éthanol, d'acétonitrile et d'isopropyléther. Un séchage à l'air donne 77 mg du complexe dont la structure, telle que résolue par diffraction des rayons X, est montrée sur la figure 5, sous la forme d'un produit jaune (Rdt : 64%).
**RMN $^1$H NMR (400 MHz, MeOD) δ ppm** : 15.74 (d, J = 8.21 Hz, 2H), 12.29 (t, J = 8.03, 8.03 Hz, 2H), 5.62 (d, J = 4.37 Hz, 1H), 5.39 (d, J = 7.81 Hz, 2H), 4.20 (d, J = 4.32 Hz, 1H), 3.16 (q, J = 7.27, 7.27, 7.25 Hz, 3H), 1.27 (t, J = 7.32, 7.32 Hz, 4.5H), -0.38 (s, 2H)
**Analyse élémentaire** : calculé (trouvé) pour $[Eu(TPDTZTB)_2]NHEt_3 \cdot 1MeOH \cdot 1{,}5H_2O$ :

C : 42,96% (42,83%)
H : 3, 16% (2, 99%)
N : 23,52% (23,46%)

**Diffraction des rayons X :**
Système cristallin monoclinique
Groupe d'espace P2(1)/c
Paramètres de maille :

a = 20,964 (8) Å α = 90°
b = 19,285 (8) Å β = 105, 007 (7) °
c = 14,278 (5) Å γ = 90°

indices R (toutes données) R1 = 0,1103

**3.3. <u>Synthèse d'un complexe de TPDTZTB et de néodyme</u>**

94,48 mg (0,178 mmol) de **TPDTZTB** en suspension dans 3 mL de méthanol sont traités avec 50 μL de TEA et soumis aux ultrasons. Puis, 52,64 mg de triflate de néodyme dans 2 mL de méthanol sont ajoutés. Un précipité jaune orangé se forme instantanément qui est complètement redissous en 1 minute environ par chauffage. La solution résultante est filtrée et laissée toute une nuit à température ambiante. On obtient ainsi de fins cristaux que l'on récupère par filtration, que l'on lave avec un faible volume d'éthanol, d'acétonitrile et d'isopropyléther.

On obtient ainsi 67,8 mg d'un produit jaune (Rdt : 58%).

**RMN $^1$H (400 MHz, MeOD)** $\delta$ **ppm :** 16.51 (s, 2H), 11.70 (d, J = 7.46 Hz, 2H), 11.53 (d, J = 3.20 Hz, 1H), 8.97 (d, J = 3.25 Hz, 1H), 5.83 (t, J = 6.10, 6.10 Hz, 2H), 3.16 (q, J = 7.32, 7.32, 7.31 Hz, 3H), 1.91 (d, J = 6.54 Hz, 2H), 1.26 (t, J = 7.31, 7.31 Hz, 4.5H)
**Analyse élémentaire :** calculé (trouvé) pour $[Nd(TPDTZTB)_2]NHEt_3 \cdot 1MeOH$ :

C : 44,08% (44,18%)
H : 3,02% (2, 96%)
N : 24,13% (24,11%)

**<u>EXEMPLE 4</u> : Complexes de 1,4,7-Tris[6-tétrazolyl-pyridin-2-yl)méthyl]-1,4,7-triazacyclononane et de divers lanthanides (europium, terbium, néodyme, gadolinium et ytterbium)**

**4.1. <u>Synthèse du 1,4,7-Tris[6-tétrazolylpyridin-2-yl)méthyl]-1,4,7-triazacyclononane</u>**

Le composé titre, noté ci-après **$H_3$TTPTCN,** qui correspond au composé de formule particulière (II-i) représentée ci-avant, est synthétisé en partant de la 2-cyano-6-méthylpyridine, notée ci-après composé **6,** selon le schéma réactionnel suivant :

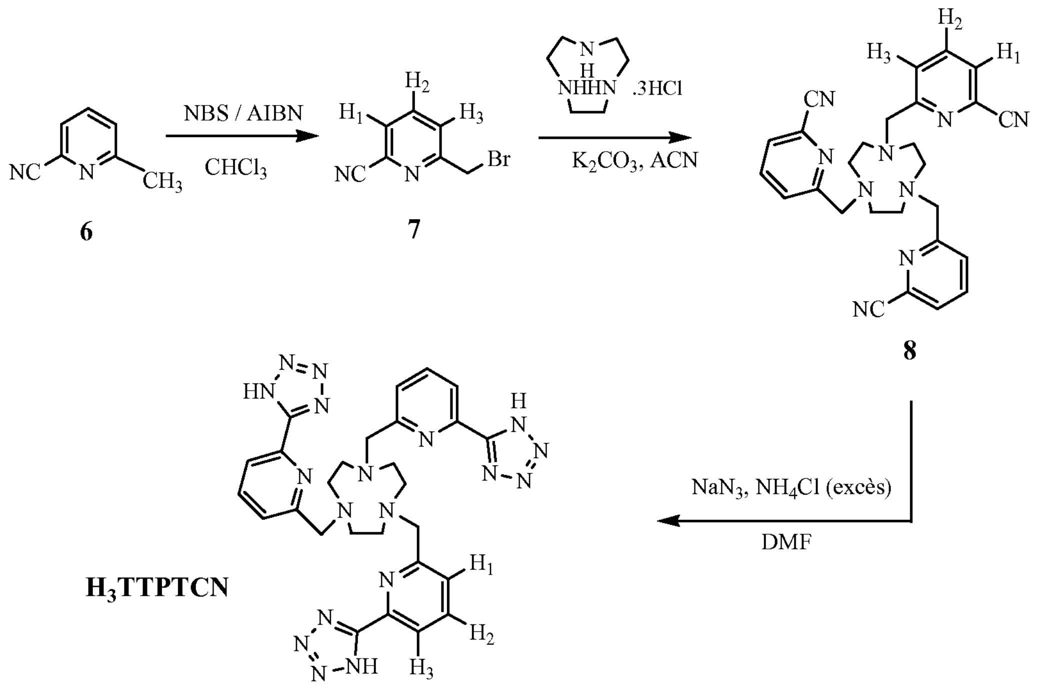


Le composé **6** est commercialement disponible.

* *Synthèse* de *la 2-bromométhyl-6-cyanopyridine ou composé* ***7***

A une solution de 400 mg (3,39 mmol) de 2-cyano-6-méthylpyridine dans 12 mL de $CHCl_3$ sont ajoutés 723 mg (4,06 mmol) de N-bromosuccinimide (NBS) et 28 mg (0,17 mmol) d'azo-bis-isobutyronitrile (AIBN). La suspension brun-orangé résultante est mise à reflux et irradiée avec une lampe UV pendant 4 heures (la réaction est suivie par CCM sur plaques de silice en utilisant du $CH_2Cl_2$ comme éluant - Rf = 0,43). Le milieu réactionnel est refroidi jusqu'à la température ambiante et le solvant est évaporé sous pression réduite. Le produit brut est purifié par chromatographie sur colonne de gel de silice en utilisant comme éluant, un gradient continu allant d'un mélange $CH_2Cl_2$/heptane 85/15 v/v à du $CH_2Cl_2$ pur.
On obtient ainsi 290 mg du composé **7** sous la forme d'une poudre microcristalline blanche (Rdt : 43%).

**RMN $^1$H (200 MHz, $CDCl_3$)** $\delta$ **ppm :** 4.55 (s, 2H, $pyCH_2Br$), 7.63 (dd, J = 7.63, 1.27 Hz, 1H, $H_3$), 7.70 (dd, J = 7.95, 1.27 Hz, 1H, $H_1$), 7.87 (t, J = 7.95Hz, 7.63Hz, 1H, $H_2$).

**Analyse élémentaire** : calculé (trouvé) :

C : 42,67% (42,80%)
H : 2, 56% (2, 57%)
N : 14,22% (14,53%)

** Synthèse du 1,4,7-Tris[6-cyanopyridin-2-yl)méthyl]-1,4,7-triazacyclononane ou composé **8***

A une suspension de 238 mg (1 mmol) de trischlorhydrate de 1,4,7-triazacyclononane dans 30 mL d'acétonitrile (ACN) anhydre, sont ajoutés successivement 853 mg (6,2 mmol) de $K_2CO_3$ et 610 mg (3,1 mmol) de composé **7** sous atmosphère d'argon. Le milieu réactionnel est mis à reflux pendant 6 heures. Après filtration des sels inorganiques et évaporation du solvant, le produit brut est purifié par chromatographie sur colonne d'alumine (activité III) en utilisant comme éluant, du $CH_2Cl_2$ pur puis un mélange $CH_2Cl_2$/EtOH 99/1 v/v.

On obtient ainsi 339 mg de composé **8** sous la forme d'une huile jaune brun (Rdt : 71%).

**RMN $^1$H NMR (200 MHz, $CDCl_3$) $\delta$ ppm :** 2.91 (s, 12H, $N(CH_2)_2N$), 3. 90 (s, 6H, py$CH_2$N), 7.63 (dd, J = 7.31, 1.27 Hz, 3H, $H_3$), 7.77 (d, J = 7.00 Hz, 3H, $H_1$), 7. 86 (d, 7.63 Hz, 3H, $H_2$)

** Synthèse du **$H_3$TTPTCN***

A une suspension de 835 mg (12,8 mmol) de $NaN_3$ et de 693 mg (12,9 mmol) de $NH_4Cl$ anhydre dans 4 mL de DMF est ajoutée une solution of 409 mg (0,85 mL) de composé **8** sous atmosphère d'argon. Le milieu réactionnel est mis à reflux toute une nuit. Les sels inorganiques sont filtrés. Le filtrat orange est mis de coté et les sels sont lavés avec 4 x 10 mL de diméthylsulfoxyde (DMSO). La fraction de DMSO jaune pâle est évaporée sous pression réduite et 5 mL d'acide chlorhydrique dilué (1 M) sont ajoutés au résidu, ce qui conduit à la formation d'un précipité. Ce précipité est récupéré par filtration, lavé à l'eau (2 x 2 mL) puis à l'éthanol (2 x 2 mL) et séché sous vide.

On obtient ainsi 280 mg de **$H_3$TTPTCN** sous la forme d'une poudre beige (Rdt : 54%).

**RMN $^1$H (200 MHz, $D_2O$) $\delta$ ppm** : 2.49 (s, 12H, $N(CH_2)_2N$), 3.46 (s, 6H, py$CH_2$N), 7.04 (d, J = 7.73 Hz, 3H, $H_3$), 7.46 (t, J = 7.70, 7.60 Hz, 3H, $H_1$), 7.57 (d, J = 7.57, 3H, $H_1$).
**RMN $^1$H (200 MHz, DMSO) $\delta$ ppm** : 7.60 (d, 3H, J = 7.4 Hz), 7.49 (t, 3H, J = 7.8, 7.4 Hz), 7.04 (d, 3H, J = 7.8 Hz), 3.47 (s, 6H), 2.48 (s, 12H)
**ES-MS *m/z* (%):** 607.14 (100) $[M + H]^+$, 623.3 (20) $[M + 2H]^{2+}$.
**Analyse élémentaire :** calculé (trouvé) pour $H_3TTPTCN \cdot 2{,}5H_2O \cdot 2HCl$ :

C : 44,75% (44,80%)
H : 5, 15% (5, 11%)
N : 34,79% (34,34%)

### 4.2. Synthèse des complexes de $H_3$TTPTCN et de lanthanides

A une solution de 29,6 mg (0,05 mmol) de triflate d'europium dans 1 mL d'eau est ajoutée une solution de 36 mg (0,05 mmol) de **$H_3$TTPTCN** dans 2 mL d'eau dont le pH est ajusté à 7 par addition d'une solution aqueuse de NaOH (1 M puis 0,1 M). Un précipité blanc se forme immédiatement. La suspension est agitée pendant 15 minutes puis le précipité est récupéré par filtration et séché sous vide.

Le complexe est isolé sous la forme $[Eu(TTPTCN)] \cdot 5{,}7H_2O$ (Rdt : 37%). Sa structure, telle que résolue par diffraction des rayons X, est montrée sur la figure 6.

**Analyse élémentaire** : calculé (trouvé) :

C : 37,78% (38,23%)
H : 4, 51% (4, 49%)
N : 29, 38% (28, 95%)

### 4.3. Synthèse d'un complexe de $H_3$TTPTCN et de terbium

A une solution de 30,1 mg (0,05 mmol) de triflate de terbium dans 1 mL d'eau est ajoutée une solution de 36 mg (0,05 mmol) de **$H_3$TTPTCN** dans 2 mL d'eau dont le pH est ajusté à 7 par addition d'une solution aqueuse de NaOH.

On procède ensuite comme décrit au point 4.2 ci-avant.

On isole ainsi un complexe [Tb(TTPTCN)]·$H_2O$ (Rdt : 55 %).

**Analyse élémentaire** : calculé (trouvé) :

C : 41,55% (41,54%)
H : 3, 74% (3, 74%)
N : 32, 30% (32,08%)

### 4.4. Synthèse d'un complexe de $H_3$TTPTCN et de néodyme

A une solution de 29,6 mg (0,05 mmol) de triflate de néodyme dans 1 mL d'eau est ajoutée une solution de 36,2 mg (0,05 mmol) de **$H_3$TTPTCN** dans 2 mL d'eau dont le pH est ajusté à 7 par addition d'une solution aqueuse de NaOH. On procède ensuite comme décrit au point 4.2 ci-avant.

On isole ainsi un complexe [Nd(TTPTCN)]·5$H_2O$ (Rdt : 42 %).

**Analyse élémentaire** : calculé (trouvé) :

C : 38,70% (39,00%)
H : 4, 45% (4, 32%)
N : 30, 09% (29, 65%)

### 4.5. Synthèse d'un complexe de $H_3$TTPTCN et de gadolinium

A une solution de 12,1 mg (0,02 mmol) de triflate de gadolinium dans 1 mL d'eau est ajoutée une solution de 12,4 mg (0,02 mmol) de **$H_3$TTPTCN** dans 1 mL d'eau dont le pH est ajusté à 7 par addition d'une solution aqueuse de NaOH. On ajoute alors environ 7 ml d'eau pour obtenir une solution limpide, on laisse ensuite s'évaporer la solution et des cristaux sont obtenus.

On isole ainsi un complexe [Gd(TTPTCN)]·6$H_2O$.

### 4.6. Synthèse d'un complexe de $H_3$TTPTCN et d'ytterbium

A une solution de 30,5 mg (0,05 mmol) de triflate d'ytterbium dans 1 mL d'eau est ajoutée une solution de 36 mg (0,05 mmol) de **$H_3$TTPTCN** dans 2 mL d'eau dont le pH est ajusté à 7 par addition d'une solution aqueuse de NaOH. On procède ensuite comme décrit au point 4.2 ci-avant.

On isole ainsi un complexe [Yb(TTPTCN)]·6,5$H_2O$ (Rdt : 30 %).

**Analyse élémentaire :** calculé (trouvé) :

C : 33, 55% (33,69%)
H : 4, 38% (4, 37%)
N : 26, 08% (25,61%)

## EXEMPLE 5 : PROPRIETES PHOTOPHYSIQUES DES COMPLEXES DE LANTHANIDES SELON L'INVENTION

### 5.1. En solution :

Les propriétés photophysiques que présentent en solution le complexe d'europium synthétisé dans l'exemple 1 ci-avant (qui a pour ligand, le 2,2'-6',2"-terpyridine-6, 6"-ditétrazole ou **TPDTZ**) ont été étudiées et comparées à celles d'un complexe d'europium ayant pour ligand, le 2,2'-6'-2"-terpyridine-6,6"-dicarboxylate, noté ci-après **TPDC,** de formule :

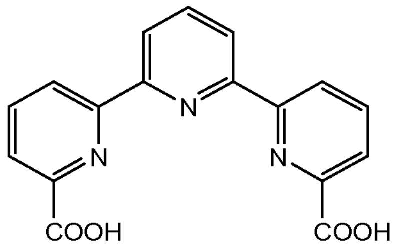

Les propriétés photophysiques en solution du complexe d'europium à base de TPDC ont été rapportées par Latva et al. dans la référence **[1].**
Le tableau 1 ci-après présente les états triplets, les durées de vie et les rendements quantiques que présentent en solution le complexe selon l'invention et le complexe étudié par Latva et al..

TABLEAU 1

| **Complexe** | **Selon l'invention** (ligand = TPDTZ) | **Etudié par Latva et al.** (ligand = TPDC) |
|---|---|---|
| Etat triplet nm ($cm^{-1}$) | 451 (22173) | 438 (22850) |
| Durée de vie (ms)<br>Rendement quantique (%) | 3,34<br>28,0 | 0,31-0,82<br>11,0 |

Ce tableau montre que le rendement quantique du complexe d'europium selon l'invention en solution est nettement plus élevé que celui rapporté par Latva et al. pour le complexe correspondants à base de TPDC.

**5.2. A l'état solide** :

Les propriétés photophysiques que présentent à l'état solide les complexes d'europium et de néodyme synthétisés dans les exemples 1 et 3 ci-avant (qui ont pour ligands, respectivement le 2,2'-6',2"-terpyridine-6,6"-ditétrazole ou **TPDTZ,** et le 4'-(5-bromo-2-thiényl)-2,2'-6'-2"-terpyridine-6,6"-ditétrazole ou **TPDTZTB**), ainsi que le complexe d'europium synthétisé dans l'exemple 2 ci-avant (qui a pour ligand, le 2, 2'-6', 2"-terpyridine-4'- (p-bromophényl)-6,6"-ditétrazole ou **TPDTZPB**) ont été étudiées et comparées les unes aux autres.
Le tableau 2 ci-après présente les états triplets, les maxima d'excitation, les durées de vie et les rendements quantiques de ces complexes à l'état solide.

TABLEAU 2

| **Ligand** | **TPDTZ** | | **TPDTZPB** | **TPDTZTB** |
|---|---|---|---|---|
| Etat triplet nm ($cm^{-1}$) | 451 (22173) | | 446 (22422) | 542 (18450) |
| Métal complexé | Eu | Nd | Eu | Nd |
| Maximum d'excitation (nm)<br>Durée de vie (ms)<br>Rendement quantique (%) | 369<br>2,50<br>35, 3 (2) | 370<br>-<br>0,22(1) | 367<br>2,47<br>29, 4 (6) | 378<br>-<br>0,29(2) |

Ce tableau montre que les rendements quantiques des complexes d'europium selon l'invention à l'état solide sont élevés, ce qui signifie que les ligands terpyridine-tétrazoles selon l'invention sont capables de sensibiliser très efficacement l'europium.
Ces ligands sont également capables de sensibiliser efficacement le néodyme comparativement aux ligands de l'art antérieur puisque les rendements quantiques obtenus pour les complexes de ce lanthanide selon l'invention sont au moins deux fois plus élevés que ceux typiquement rapportés dans la littérature pour les complexes de ce même lanthanide.
Par ailleurs, on peut noter que la valeur de temps de vie obtenue pour le complexe de **TPDTZ** et d'europium à l'état solide est peu éloigné de celle obtenue pour ce même complexe en solution (voir tableau 1), ce qui plaide en faveur d'une très bonne stabilité des complexes selon l'invention.
Ont également été testées les propriétés photophysiques que présentent à l'état solide les complexes d'europium et de terbium synthétisés dans l'exemple 4 ci-avant et qui ont pour ligand, le 1,4,7-Tris[6-tétrazolylpyridin-2-yl)méthyl]-1,4,7-triazacyclononane ou **$H_3$TTPTCN.**

Les résultats de ces tests, en termes de rendement quantique et de temps de vie, sont présentés dans le tableau 3 ci-après.

TABLEAU 3

| Métal complexé | Eu | Tb |
|---|---|---|
| Durée de vie (ms) | 1, 13 (4) | 1, 56 (6) |
| Rendement quantique (%) | 19, 8 (4) | 56, 1 (3) |

Là également, on constate que les complexes d'europium et de terbium selon l'invention présentent à l'état solide des rendements quantiques très élevés. A titre de comparaison, le rendement quantique obtenu pour un complexe d'europium préparé à partir d'un analogue carboxylate du **$H_3$TTPTCN,** à l'état solide, n'est que de 6%.

En outre, comme le montre la figure 7 qui représente les spectres d'excitation électronique que présentent à l'état solide le complexe d'europium synthétisé dans l'exemple 1 ci-avant (courbe A) et le complexe correspondant à base de TPDC (courbe B), ainsi que les spectres d'absorption d'un substrat en verre (courbe S1) et d'un substrat en ITO (courbe S2) montre que la zone de non recouvrement de ces spectres est nettement plus large dans le cas du complexe selon l'invention.

Il en résulte que ce complexe peut être excité avec des radiations moins énergétiques que celles nécessaires au complexe de l'art antérieur, par exemple à 400 nm, ce qui réduit fortement la probabilité de le dégrader.

**REFERENCES CITEES**

[1] Latva et al., Journal of Luminescence 1997, 75, 149-169

[2] Chatterton et al., Angewandte Chemie, édition internationale en anglais 2005, 44, 7595-7598

[3] Nonat et al., Chemistry, a European Journal 2006, 12, 7133-7150)

[4] Petoud et al., Journal of the American Chemical Society 2003, 125, 13324-13325

[5] Moore et al., Inorganic Chemistry 2007, 46, 5468-5470

[6] Vögtle et al., ChemPhysChem 2001, 2, 769-773

[7] Chauvin et al., Spectroscopy Letters 2004, 37, 517-532

[8] Mello et al., Advanced Materials 1997, 9, 230-232

[9] Mukkala et al., Helvetica Chimica Acta 1992, 75, 1621-1632

[10] Hovinen et al., Organic Letters 2001, 3, 2473-2476

[11] López et al., Inorganica Chimica Acta 2004, 357, 3525-3531

**Revendications**

1. Utilisation d'un composé répondant à la formule générale (II) ci-après :

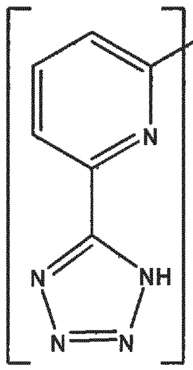

(II)

dans laquelle :

Y représente un cycle pyridine et n est égal à 1, moyennant quoi le composé répond à la formule (III) ci-après :

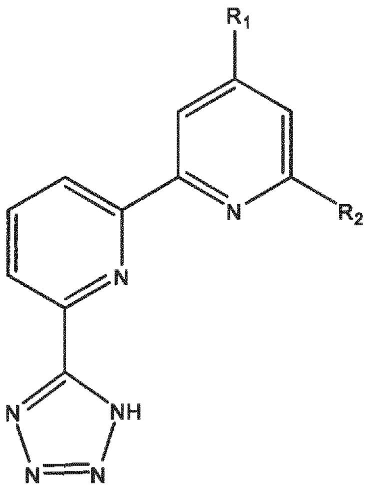

(III)

dans laquelle :

$R_1$ représente un atome d'hydrogène ou d'halogène ; un cycle aromatique à 5 ou 6 chaînons ou un enchaînement de plusieurs cycles aromatiques à 5 ou 6 chaînons liés les uns aux autres par une liaison covalente, ce cycle ou l'un au moins de ces cycles comportant éventuellement un ou plusieurs hétéroatomes et/ou substituants ; ou un groupe hydrocarboné linéaire ou ramifié en $C_1$ à $C_{12}$ comportant éventuellement un ou plusieurs hétéroatomes ; et

$R_2$ représente un groupe acide carboxylique ; un cycle aromatique à 5 ou 6 chaînons, comportant éventuellement un ou plusieurs hétéroatomes et/ou substituants ; ou un motif de formule (I) ci-après :

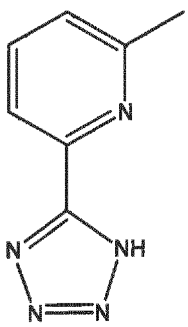

(I) ;

ou bien

Y représente un groupe de formule $-(CH_2)_p-Z$ où p est égal à 1 ou 2, Z représente un cycle non aromatique de 5 à 9 chaînons, qui comporte éventuellement un ou plusieurs hétéroatomes et/ou substituants, et n est un nombre entier allant de 1 à 5 ;
le ou les substituants étant choisis parmi les atomes d'halogène et les groupes -COOR', -CHO, -OR', -SR', -SCOR', $-SO_2R'$, -NR'R", -CONR'R", $-C(Hal)_3$, $-OC(Hal)_3$, -C(O)Hal et -CN dans lesquels R' et R" représentent un atome d'hydrogène ou un groupe alkyle en $C_1$ à $C_3$, tandis que Hal représente un atome d'halogène ;

comme chromophore organique de complexation d'un lanthanide.

**2.** Utilisation selon la revendication 1, dans laquelle $R_1$ est un groupe phényle ou thiophène éventuellement substitué par un ou plusieurs atomes d'halogène.

**3.** Utilisation selon la revendication 1, dans laquelle Z est un cycle non aromatique à 9 chaînons, comportant de 2 à 5 hétéroatomes.

**4.** Utilisation selon la revendication 3, dans laquelle Z est un cycle triazacyclononane ou tétraazacyclononane.

**5.** Utilisation selon la revendication 1, dans laquelle le composé répond à l'une quelconque des formules particulières (II-a), (II-b), (II-c), (II-d), (II-e), (II-f), (II-i) et (II-j) ci-après :

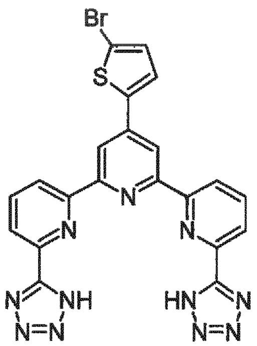

(II-a)

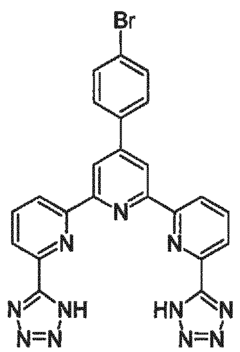

(II-b)

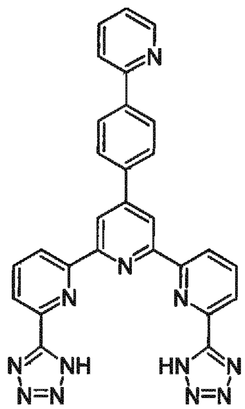

(II-c)

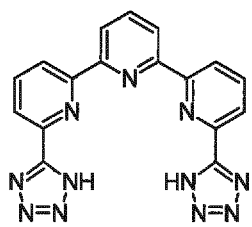

(II-d)

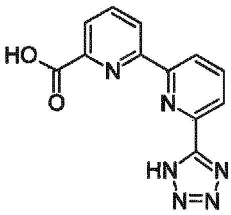

(II-e)

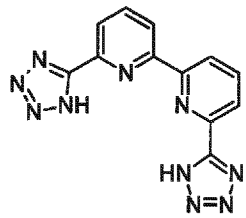

(II-f)

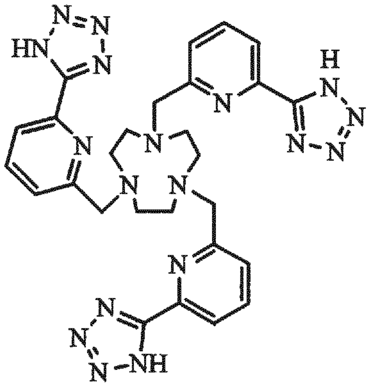

(II-i)

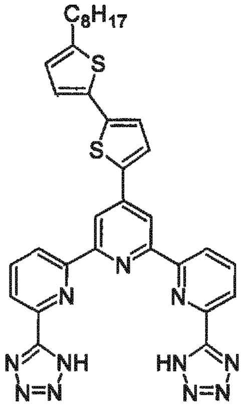

(II-j)

6. Utilisation selon l'une quelconque des revendications précédentes, dans laquelle le lanthanide est choisi parmi l'europium, le terbium et le néodyme.

7. Complexe d'un composé tel que défini dans l'une quelconque des revendications 1 à 6 et d'un lanthanide.

8. Complexe selon la revendication 7, dans lequel le composé répond à l'une des formules particulières (II-a), (II-b), (II-c), (II-d), (II-i) et (II-j) ci-après :

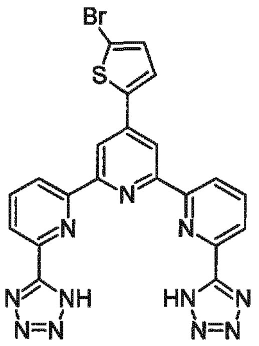

(II-a)

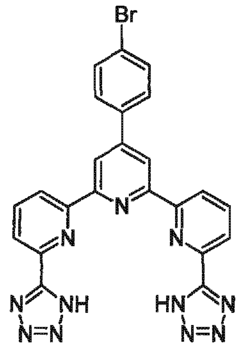

(II-b)

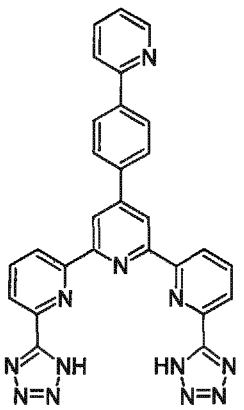

(II-c)

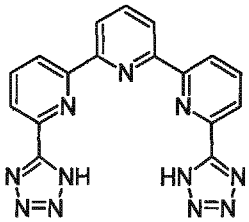

(II-d)

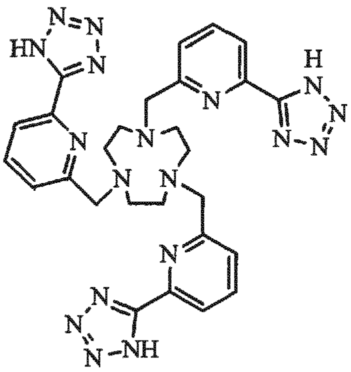

(II-i)

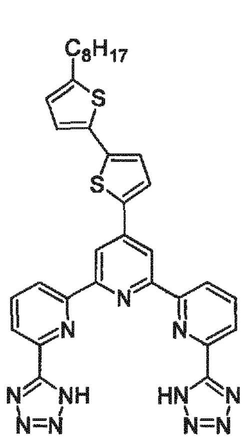

(II-j)

**9.** Complexe selon la revendication 7 ou la revendication 8, dans lequel le lanthanide est choisi parmi l'europium, le terbium et le néodyme.

**10.** Composé, qui répond à la formule générale (II) ci-après :

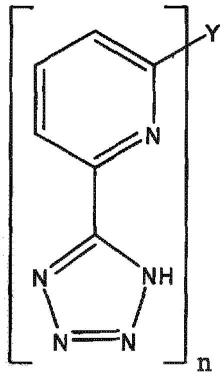

(II)

dans laquelle :

Y représente un cycle pyridine et n est égal à 1, moyennant quoi le composé répond à la formule (III) ci-après :

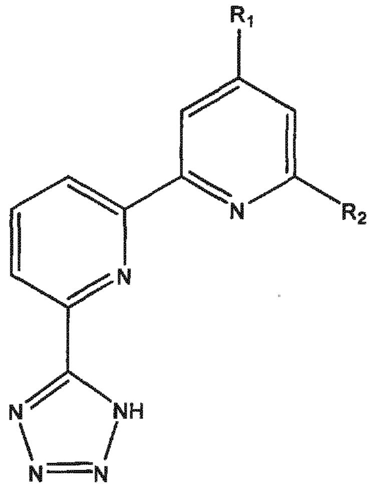

(III)

dans laquelle :

$R_1$ représente un atome d'hydrogène ou d'halogène ; un cycle aromatique à 5 ou 6 chaînons ou un enchaînement de plusieurs cycles aromatiques à 5 ou 6 chaînons liés les uns aux autres par une liaison covalente, ce cycle ou l'un au moins de ces cycles comportant éventuellement un ou plusieurs hétéroatomes et/ou substituants ; ou encore un groupe hydrocarboné linéaire ou ramifié en $C_1$ à $C_{12}$ comportant éventuellement un ou plusieurs hétéroatomes ; et

$R_2$ représente un groupe acide carboxylique ; un cycle aromatique à 5 ou 6 chaînons, comportant éventuellement un ou plusieurs hétéroatomes et/ou substituants ; ou bien un motif de formule (I) ci-après :

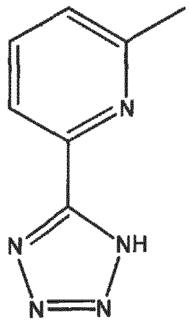

(I) ;

ou bien

Y représente un groupe de formule $-(CH_2)_p-Z$ où p est un nombre entier égal à 1 ou 2, Z représente un cycle non aromatique de 5 à 9 chaînons, qui comporte éventuellement un ou plusieurs hétéroatomes et/ou substituants, et n est un nombre entier allant de 1 à 5 ;

le ou les substituants étant choisis parmi les atomes d'halogène et les groupes -COOR', -CHO, -OR', -SR', -SCOR', $-SO_2R'$, -NR'R", -CONR'R", $-C(Hal)_3$, $-OC(Hal)_3$, -C(O)Hal et -CN dans lesquels R' et R" représentent un atome d'hydrogène ou un groupe alkyle en $C_1$ à $C_3$, tandis que Hal représente un atome d'halogène.

**11.** Composé selon la revendication 10, dans lequel $R_1$ est un groupe phényle ou thiophène éventuellement substitué par un ou plusieurs atomes d'halogène.

**12.** Composé selon la revendication 10, dans lequel Z est un cycle non aromatique à 9 chaînons, comportant de 2 à 5 hétéroatomes.

**13.** Composé selon la revendication 12, dans lequel Z est un cycle triazacyclononane ou tétraazacyclononane.

**14.** Composé selon l'une quelconque des revendications 10 à 13, qui répond à l'une quelconque des formules particulières (II-a), (II-b), (II-c), (II-d), (II-e), (II-f), (II-i) et (II-j) ci-après :

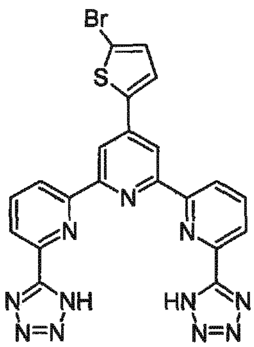

(II-a)

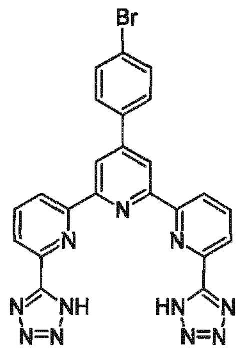

(II-b)

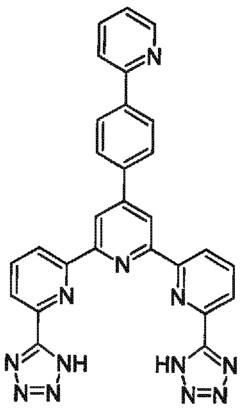

(II-c)

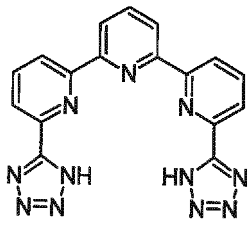

(II-d)

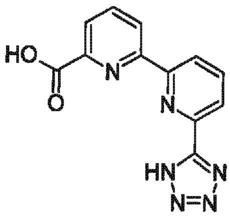

(II-e)

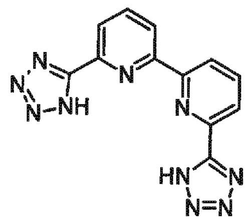

(II-f)

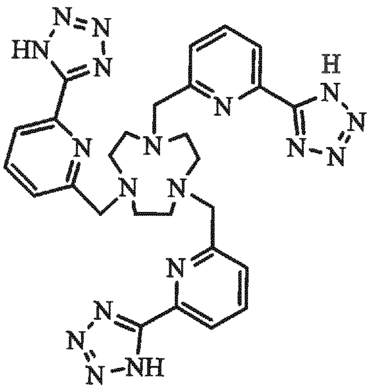

(II-i)

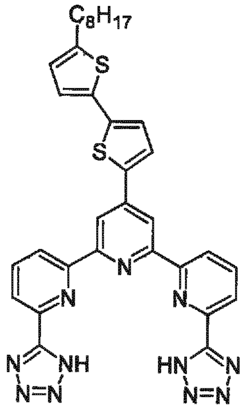

(II-j)

**Patentansprüche**

1. Verwendung einer Verbindung, die der folgenden allgemeinen Formel (II) entspricht:

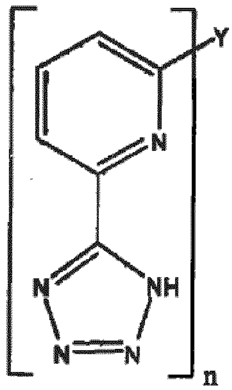

(II)

wobei:

Y einen Pyridinring repräsentiert und n=1, wodurch die Verbindung der folgenden Formel (III) entspricht:

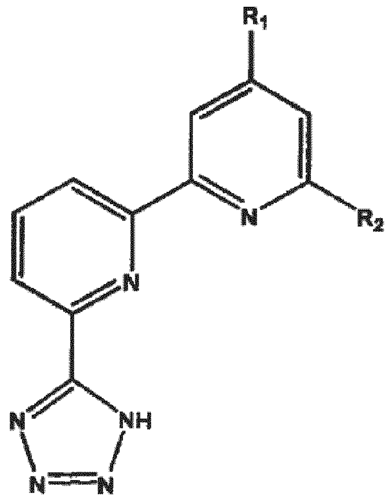

(III)

wobei:

$R_1$ repräsentiert: ein Wasserstoff- oder Halogenatom; einen aromatischen Ring mit 5 oder 6 Ringgliedern oder eine Verkettung von mehreren aromatischen Ringen mit 5 oder 6 Ringgliedern, die miteinander durch eine kovalente Bindung verbunden sind, wobei dieser Ring oder mindestens einer dieser Ringe eventuell eines oder mehrere Heteroatome und/oder Substituenten aufweist; oder eine lineare oder verzweigte Kohlenwasserstoffgruppe, und zwar $C_1$ bis $C_{12}$, die eventuell eines oder mehrere Heteroatome aufweist; und $R_2$ repräsentiert: eine Carboxylsäuregruppe; einen aromatischen Ring mit 5 oder 6 Ringgliedern, der eventuell eines oder mehrere Heteroatome und/oder Substituenten aufweist; oder eine Einheit der folgenden Formel (I):

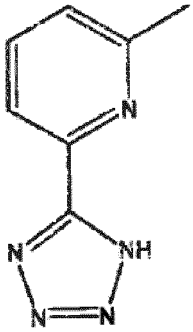

(I)

;

oder auch

Y eine Gruppe der Formel $-(CH_2)_p-Z$ repräsentiert, wobei p einen Wert von 1 oder 2 hat, Z einen nicht-aromatischen Ring mit 5 bis 9 Ringgliedern repräsentiert, der eventuell eines oder mehrere Heteroatome und/oder Substituenten aufweist, und n eine Ganzzahl im Bereich von 1 bis 5 ist;

der oder die Substituenten gewählt sind aus Halogenatomen und den Gruppen: -COOR',-CHO, -OR', -SR', -SCOR', $-SO_2R'$, -NR'R", -CONR'R", $-C(Hal)_3$, $-OC(Hal)_3$, -C(O)Hal und-CN, wobei R' und R" ein Wasserstoffatom oder eine Alkylgruppe, und zwar $C_1$ bis $C_3$, repräsentieren, hingegen Hal ein Halogenatom repräsentiert;

als organischer Chromophor zur Lanthanoidenkomplexbildung.

**2.** Verwendung nach Anspruch 1, wobei $R_1$ eine Phenyl- oder Thiophengruppe ist, die eventuell durch eines oder mehrere Halogenatome substituiert ist.

**3.** Verwendung nach Anspruch 1, wobei Z ein nicht-aromatischer Ring mit 9 Ringgliedern ist, der 2 bis 5 Heteroatome aufweist.

**4.** Verwendung nach Anspruch 3, wobei Z ein Triazacyclononanon- oder Tetraazacyclononanon-Ring ist.

**5.** Verwendung nach Anspruch 1, wobei die Verbindung einer der folgenden speziellen Formeln (II-a), (II-b), (II-c), (II-d), (II-e), (II-f), (II-i), (II-j) entspricht:

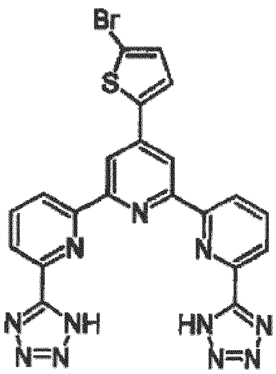

(II-a)

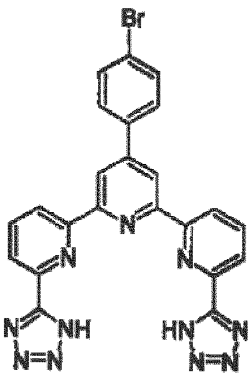

(II-b)

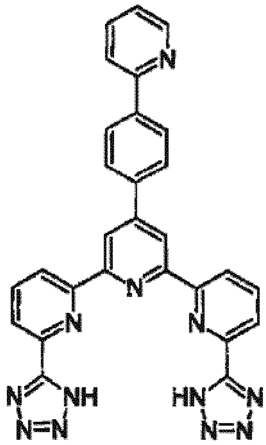

(II-c)

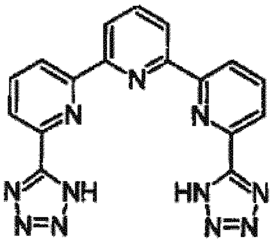

(II-d)

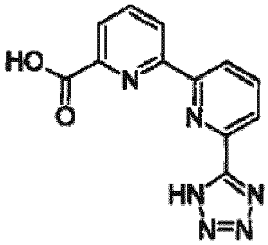

(II-e)

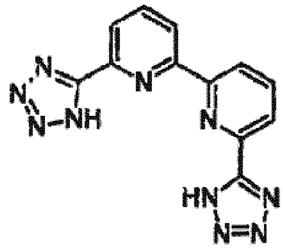

(II-f)

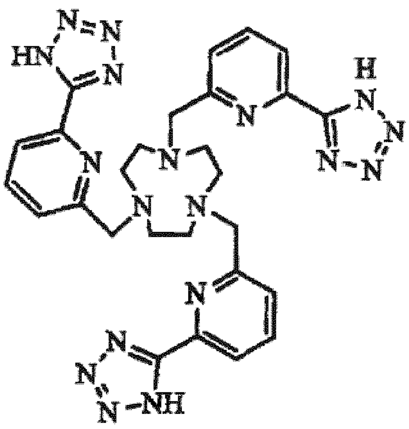

(II-i)

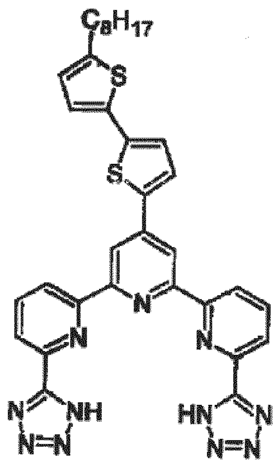

(II-j)

6. Verwendung nach einem der vorhergehenden Ansprüche, wobei das Lanthanoid gewählt ist aus Europium, Terbium und Neodym.

7. Komplex aus einer Verbindung, wie definiert in einem der Ansprüche 1 bis 6, und aus einem Lanthanoid.

8. Komplex nach Anspruch 7, wobei die Verbindung einer der folgenden speziellen Formeln (IIa), (II-b), (II-c), (II-d), (II-i), (II-j) entspricht:

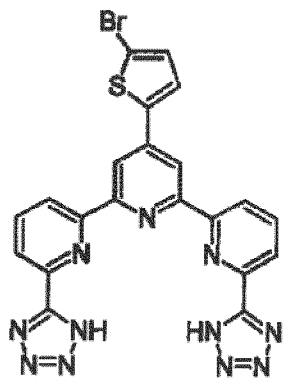

(II-a)

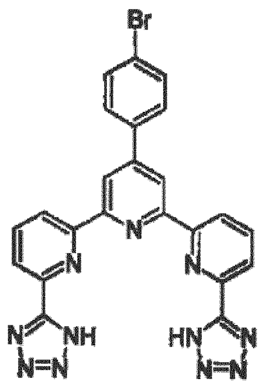

(II-b)

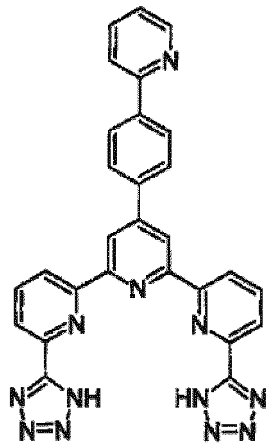

(II-c)

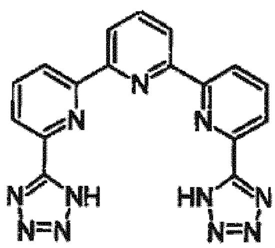

(II-d)

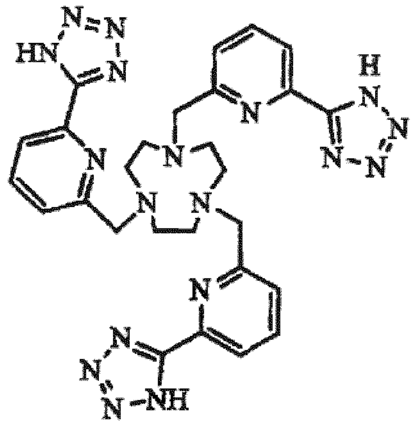

(II-i)

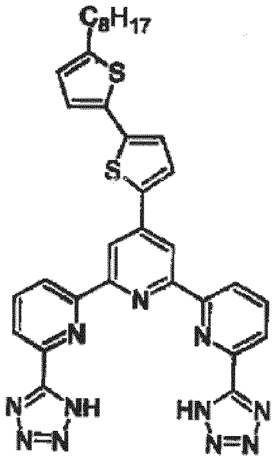

(II-j)

**9.** Komplex nach Anspruch 7 oder Anspruch 8, wobei das Lanthanoid gewählt ist aus Europium, Terbium und Neodym.

**10.** Verbindung, die der folgenden allgemeinen Formel (II) entspricht:

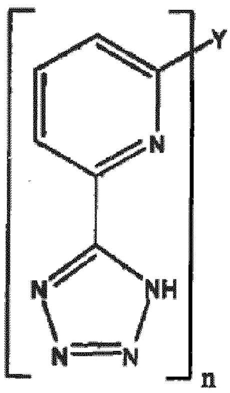

(II)

wobei:

Y einen Pyridinring repräsentiert und n=1, wodurch die Verbindung der folgenden Formel (III) entspricht:

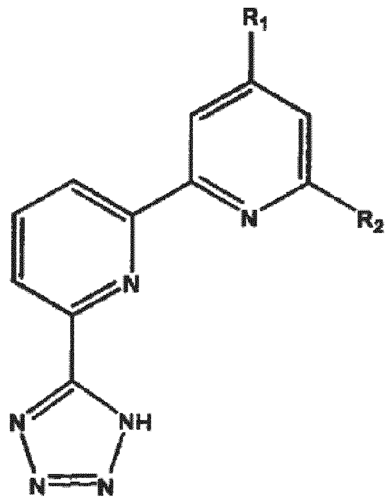

(III)

wobei:

$R_1$ repräsentiert: ein Wasserstoff- oder Halogenatom; einen aromatischen Ring mit 5 oder 6 Ringgliedern oder eine Verkettung von mehreren aromatischen Ringen mit 5 oder 6 Ringgliedern, die miteinander durch eine kovalente Bindung verbunden sind, wobei dieser Ring oder mindestens einer dieser Ringe eventuell eines oder mehrere Heteroatome und/oder Substituenten aufweist; oder auch eine lineare oder verzweigte Kohlenwasserstoffgruppe, und zwar $C_1$ bis $C_{12}$, die eventuell eines oder mehrere Heteroatome aufweist; und $R_2$ repräsentiert: eine Carboxylsäuregruppe; einen aromatischen Ring mit 5 oder 6 Ringgliedern, der eventuell eines oder mehrere Heteroatome und/oder Substituenten aufweist; oder eine Einheit der folgenden Formel (I):

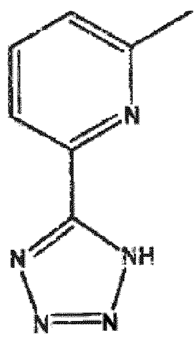

(I)

;

oder auch
Y eine Gruppe der Formel $-(CH_2)_p-Z$ repräsentiert, wobei p eine Ganzzahl mit einem Wert von 1 oder 2 ist, Z einen nicht-aromatischen Ring mit 5 bis 9 Ringgliedern repräsentiert, der eventuell eines oder mehrere Heteroatome und/oder Substituenten aufweist, und n eine Ganzzahl im Bereich von 1 bis 5 ist;
der oder die Substituenten gewählt sind aus Halogenatomen und den Gruppen: -COOR',-CHO, -OR', -SR', -SCOR', $-SO_2R'$, -NR'R", -CONR'R", $-C(Hal)_3$, $-OC(Hal)_3$, -C(O)Hal und-CN, wobei R' und R" ein Wasserstoffatom oder eine Alkylgruppe, und zwar $C_1$ bis $C_3$, repräsentieren, hingegen Hal ein Halogenatom repräsentiert.

**11.** Verbindung nach Anspruch 10, wobei $R_1$ eine Phenyl- oder Thiophengruppe ist, die eventuell durch eines oder mehrere Halogenatome substituiert ist.

**12.** Verbindung nach Anspruch 10, wobei Z ein nicht-aromatischer Ring mit 9 Ringgliedern ist, der 2 bis 5 Heteroatome aufweist.

**13.** Verbindung nach Anspruch 12, wobei Z ein Triazacyclononanon- oder Tetraazacyclononanon-Ring ist.

**14.** Verbindung nach einem der Ansprüche 10 bis 13, die einer der folgenden speziellen Formeln (II-a), (II-b), (II-c), (II-d), (II-e), (II-f), (II-i), (II-j) entspricht:

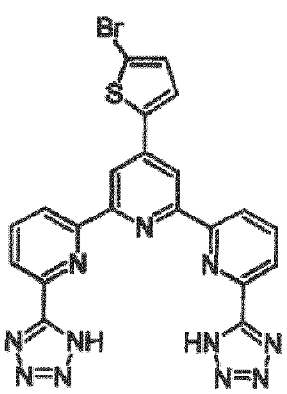

(II-a)

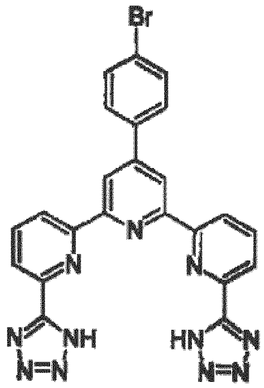

(II-b)

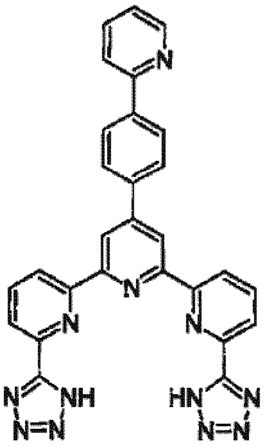

(II-c)

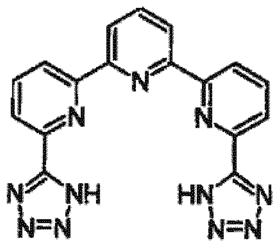

(II-d)

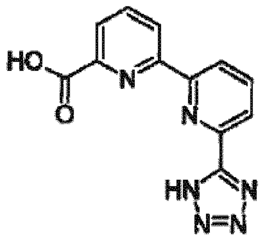

(II-e)

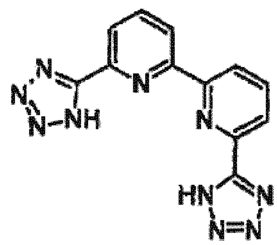

(II-f)

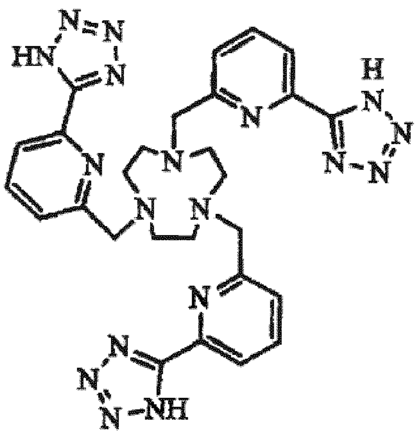

(II-i)

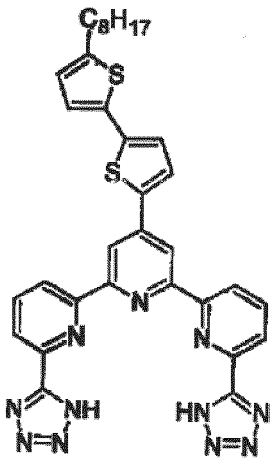

(II-j)

## Claims

**1.** Use of a compound conforming to general formula (II) below:

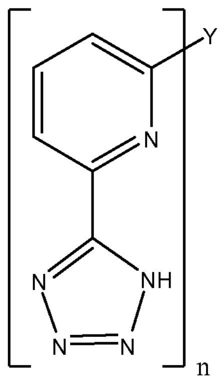

(II)

wherein:

Y represents a pyridine ring and n is 1, whereby the compound conforms to formula (III) below:

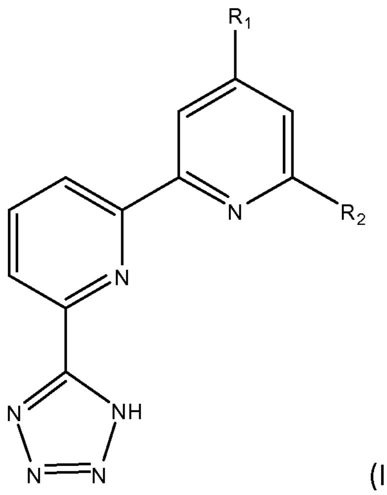

(III)

wherein:

$R_1$ represents a hydrogen or halogen atom; a 5- or 6-membered aromatic ring or a sequence of several 5- or 6-membered aromatic rings bonded to one another by a covalent bond, this ring or at least one of these rings optionally comprising one or more heteroatoms and/or substituents; or a linear or branched $C_1$ to $C_{12}$ hydrocarbon group optionally comprising one or more heteroatoms; and

$R_2$ represents a carboxylic acid group; a 5- or 6-membered aromatic ring, optionally comprising one or more heteroatoms and/or substituents; or else a unit of formula (I) below:

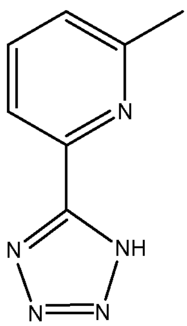

(I);

or else

Y represents a group of formula $-(CH_2)_p-Z$ where p is 1 or 2, Z is a 5- to 9-membered non-aromatic ring, which optionally comprises one or more heteroatoms and/or substituents, and n is an integer from 1 to 5; the substituent(s) being selected from the halogen atoms and the -COOR', -CHO, -OR', -SR', -SCOR', $-SO_2R'$, -NR'R", -CONR'R", $-C(Hal)_3$, $-OC(Hal)_3$, -C(O)Hal and -CN groups in which R' and R" represent a hydrogen atom or a $C_1$ to $C_3$ alkyl group, while Hal represents a halogen atom;

as an organic chromophore for complexing a lanthanide.

2. Use according to claim 1, wherein $R_1$ is a phenyl or thiophene group optionally substituted by one or more halogen atoms.

3. Use according to claim 1, wherein Z is a 9-membered non-aromatic ring, comprising 2 to 5 heteroatoms.

4. Use according to claim 3, wherein Z is a triazacyclononane or tetraazacyclononane ring.

5. Use according to claim 1, wherein the compound conforms to any one of particular formulae (II-a), (II-b), (II-c), (II-d), (II-e), (II-f), (II-i) and (II-j) below:

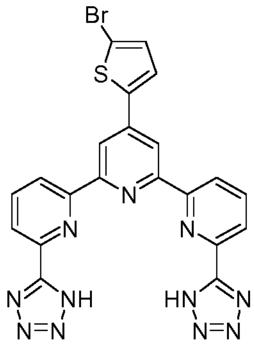

(II-a)

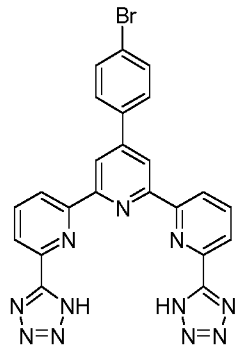

(II-b)

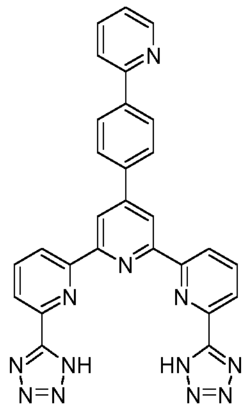

(II-c)

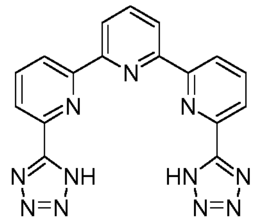

(II-d)

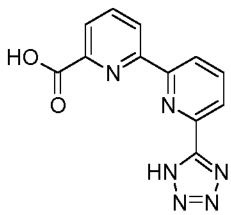

(II-e)

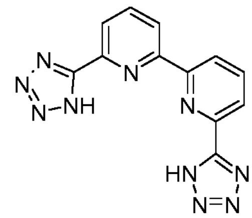

(II-f)

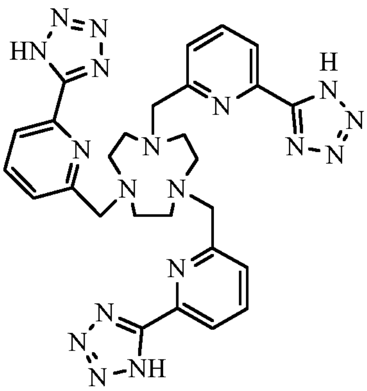

(II-i)

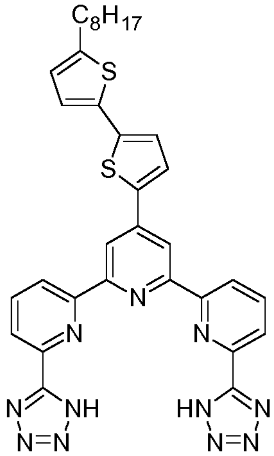

(II-j)

**6.** Use according to any one of the preceding claims, wherein the lanthanide is selected from europium, terbium and neodymium.

**7.** Complex of a compound as defined in any one of claims 1 to 6 and of a lanthanide.

**8.** Complex according to claim 7, wherein the compound conforms to one of particular formulae (II-a), (II-b), (II-c), (II-d), (II-i) and (II-j) below:

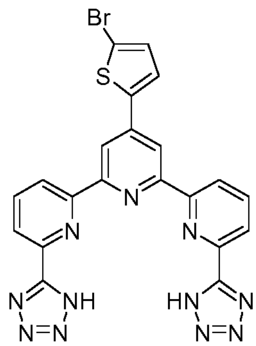

(II-a)

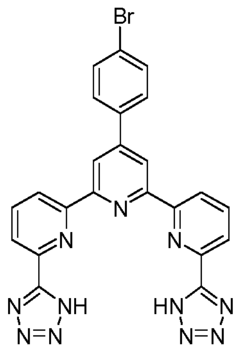

(II-b)

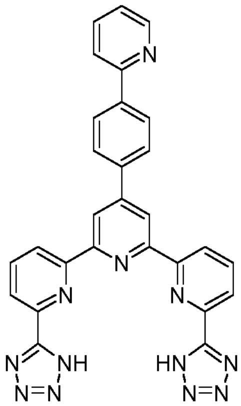

(II-c)

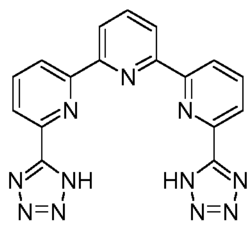

(II-d)

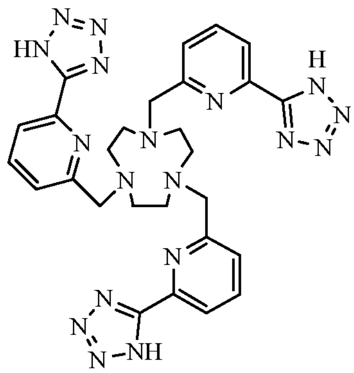

(II-i)

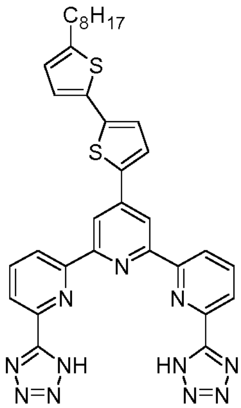

(II-j)

**9.** Complex according to claim 7 or claim 8, wherein the lanthanide is selected from europium, terbium and neodymium.

**10.** Compound conforming to general formula (II) below:

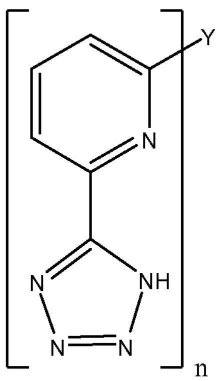

(II)

wherein:

Y represents a pyridine ring and n is 1, whereby the compound conforms to formula (III) below:

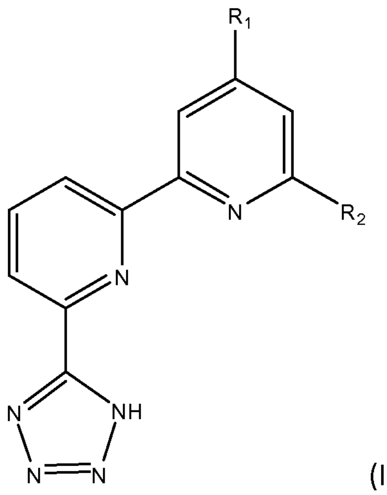

(III)

wherein:

$R_1$ represents a hydrogen or halogen atom; a 5- or 6-membered aromatic ring or a sequence of several 5- or 6-membered aromatic rings bonded to one another by a covalent bond, this ring or at least one of these rings optionally comprising one or more heteroatoms and/or substituents; or a linear or branched $C_1$ to $C_{12}$ hydrocarbon group optionally comprising one or more heteroatoms; and

$R_2$ represents a carboxylic acid group; a 5- or 6-membered aromatic ring, optionally comprising one or more heteroatoms and/or substituents; or else a unit of formula (I) below:

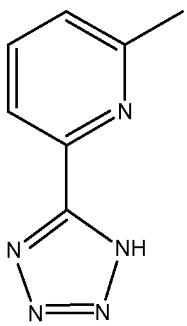

(I);

or else

Y represents a group of formula $-(CH_2)_p-Z$ where p is 1 or 2, Z is a 5- to 9-membered non-aromatic ring, which optionally comprises one or more heteroatoms and/or substituents, and n is an integer from 1 to 5; the substituent(s) being selected from the halogen atoms and the -COOR', -CHO, -OR', -SR', -SCOR', $-SO_2R'$, -NR'R", -CONR'R", $-C(Hal)_3$, $-OC(Hal)_3$, -C(O)Hal and -CN groups in which R' and R" represent a hydrogen atom or a $C_1$ to $C_3$ alkyl group, while Hal represents a halogen atom.

**11.** Compound according to claim 10, wherein Y is a phenyl or thiophene group optionally substituted by one or more halogen atoms.

**12.** Compound according to claim 10, wherein Z is a 9-membered non-aromatic ring comprising 2 to 5 heteroatoms.

**13.** Compound according to claim 12, wherein Z is a triazacyclononane or tetraazacyclononane ring.

**14.** Compound according to any one of claims 10 to 13, which conforms to any one of particular formulae (II-a), (II-b), (II-c), (II-d), (II-e), (II-f), (II-i) and (II-j) below:

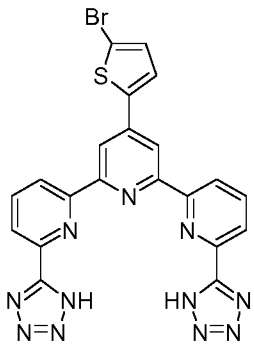

(II-a)

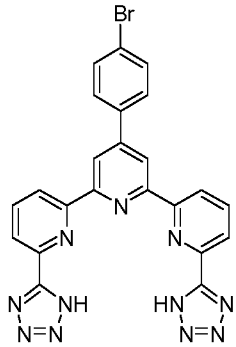

(II-b)

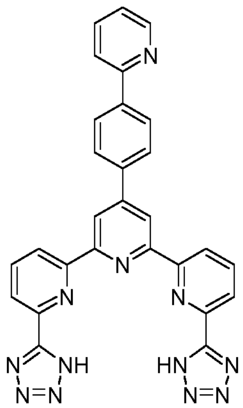

(II-c)

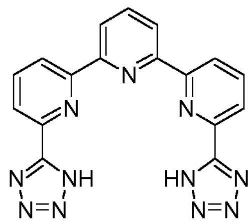

(II-d)

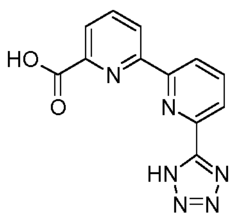

(II-e)

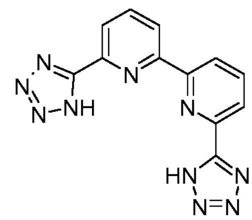

(II-f)

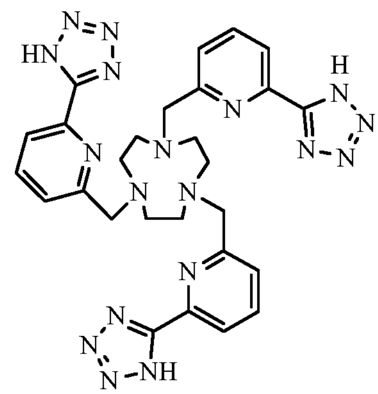

(II-i)

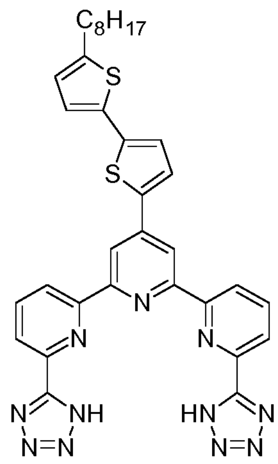

(II-j)

FIG.1

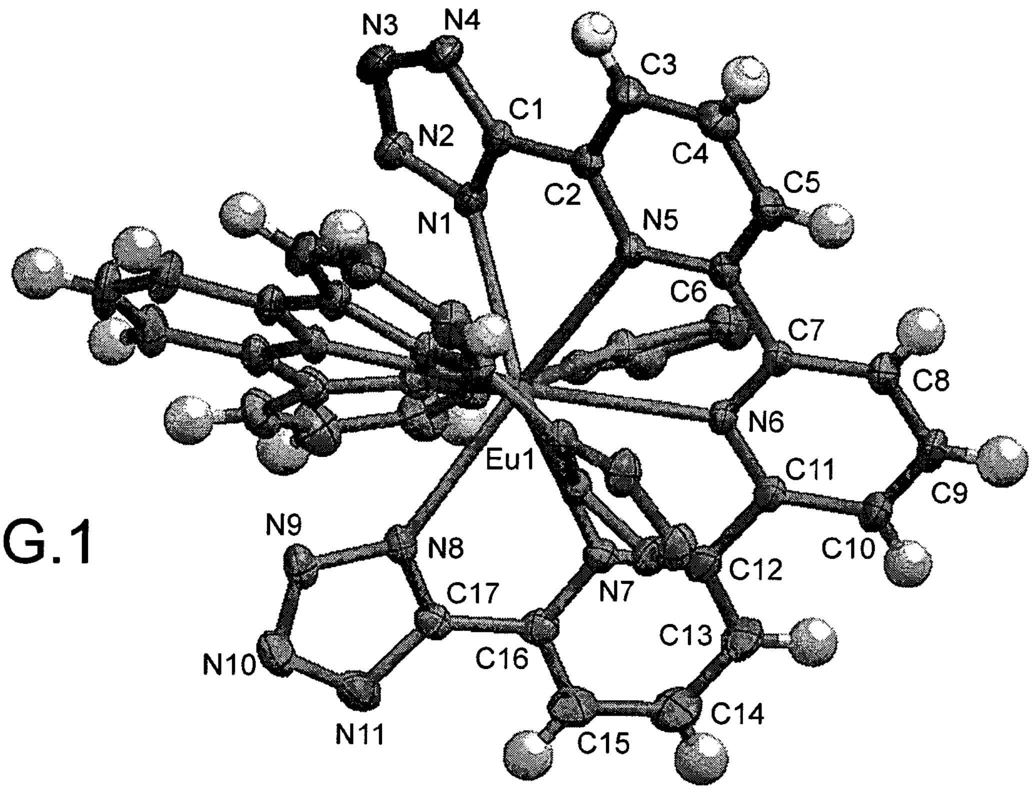

FIG.2

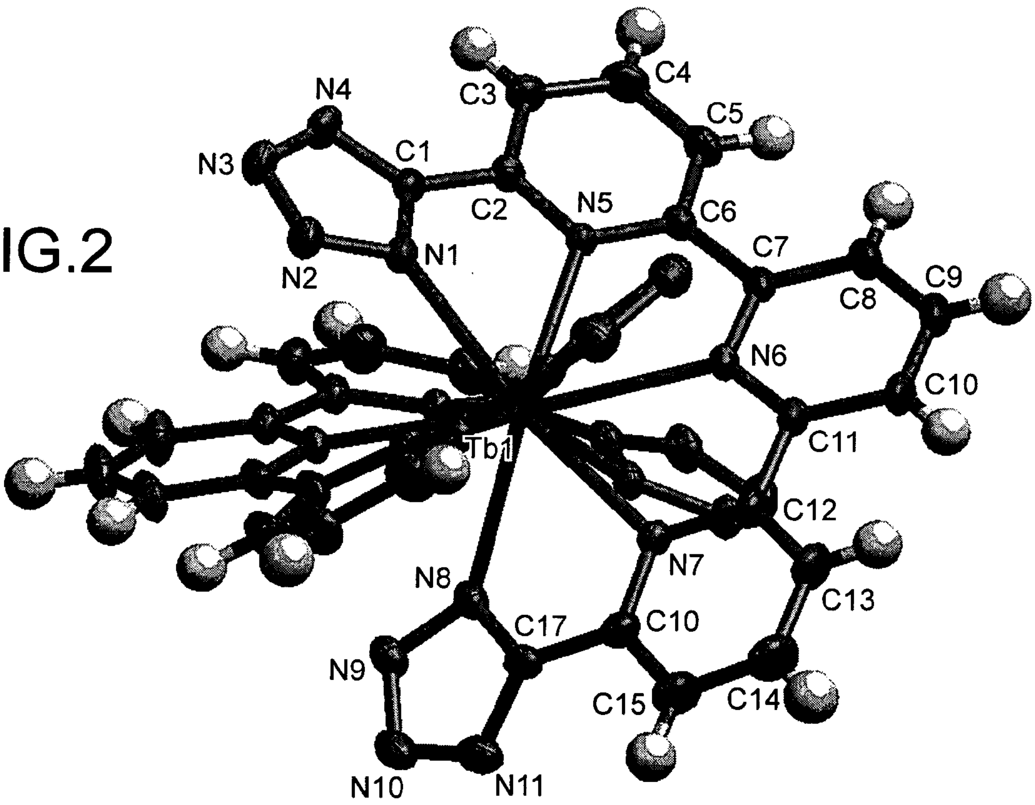

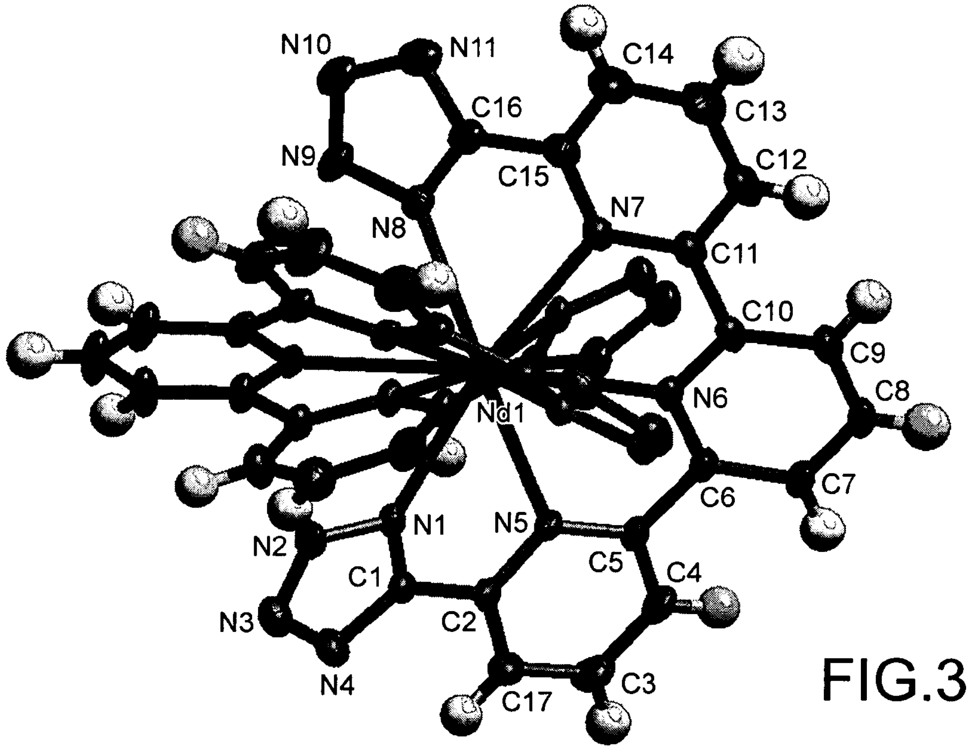

FIG.3

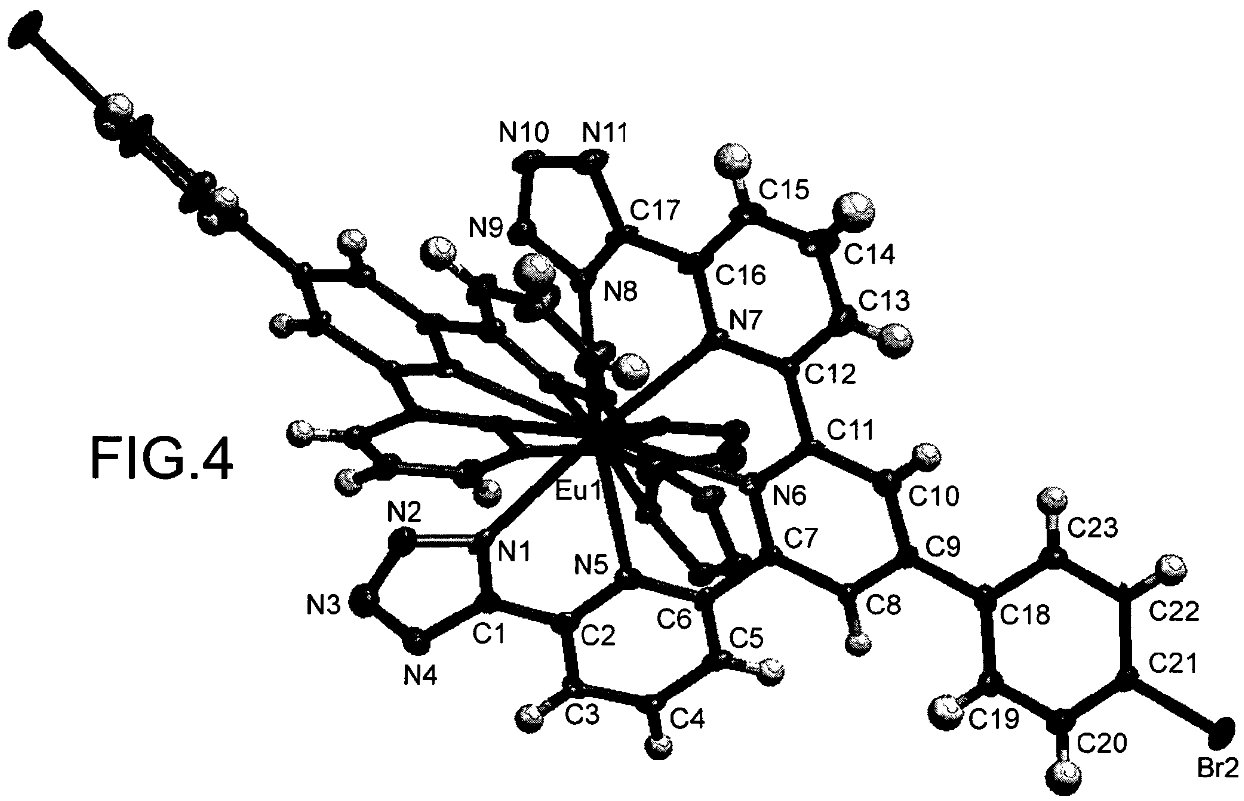

FIG.4

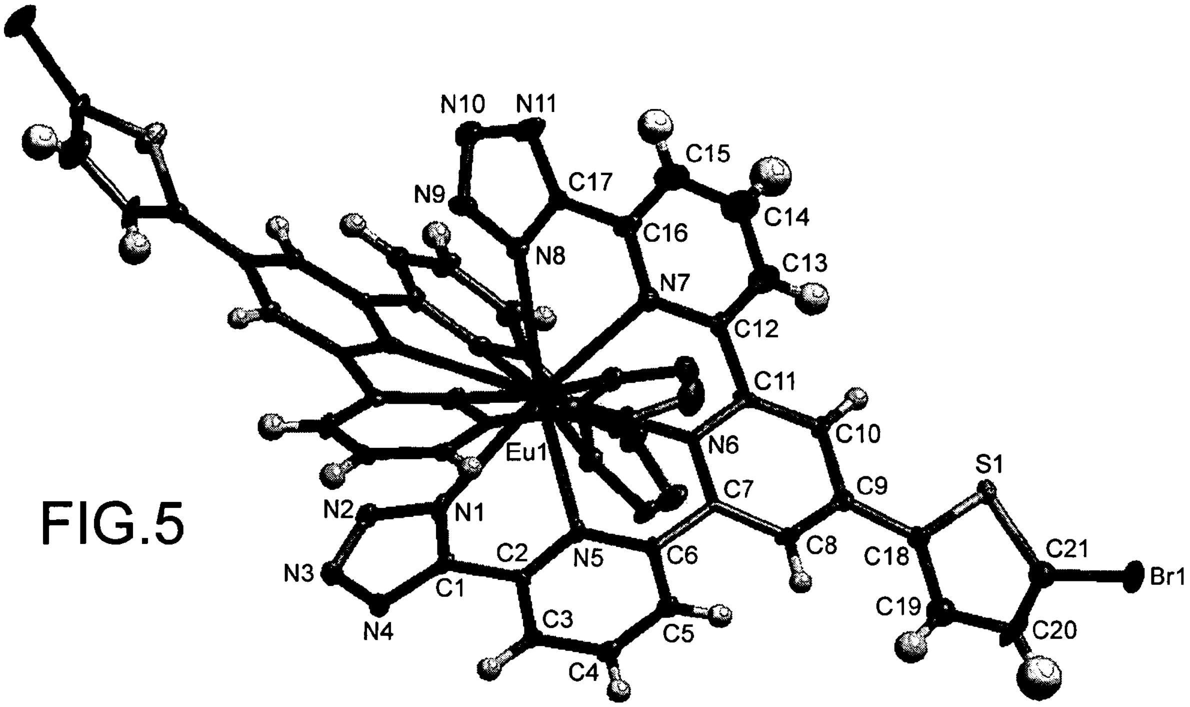

FIG.5

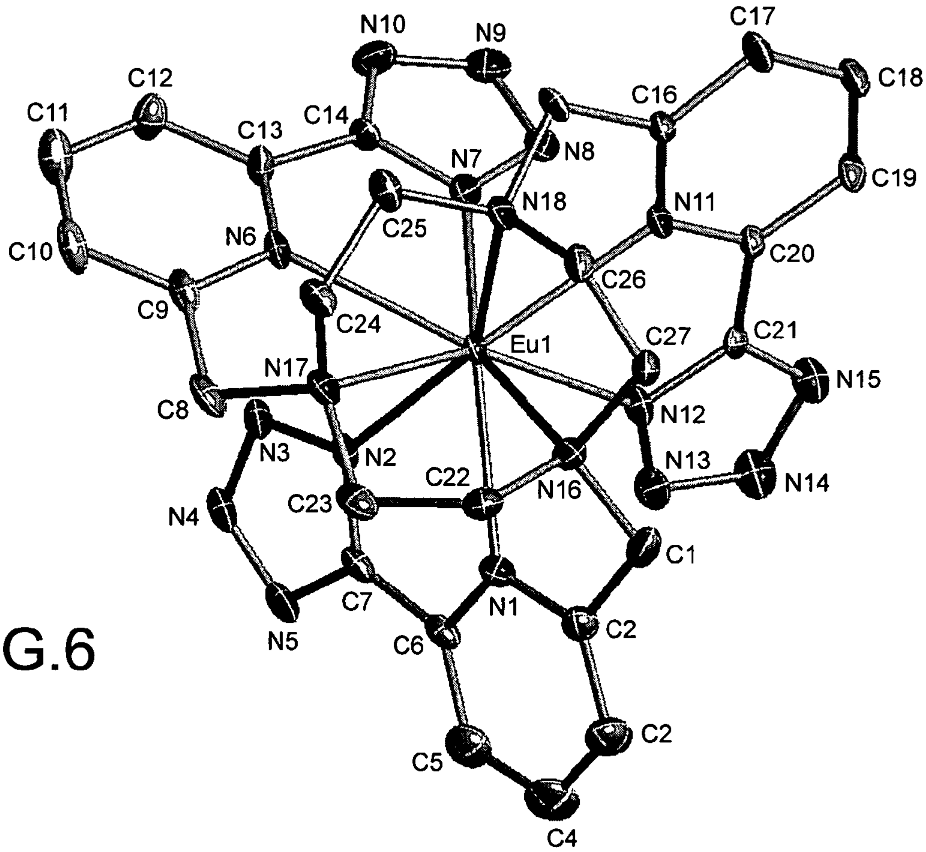

FIG.6

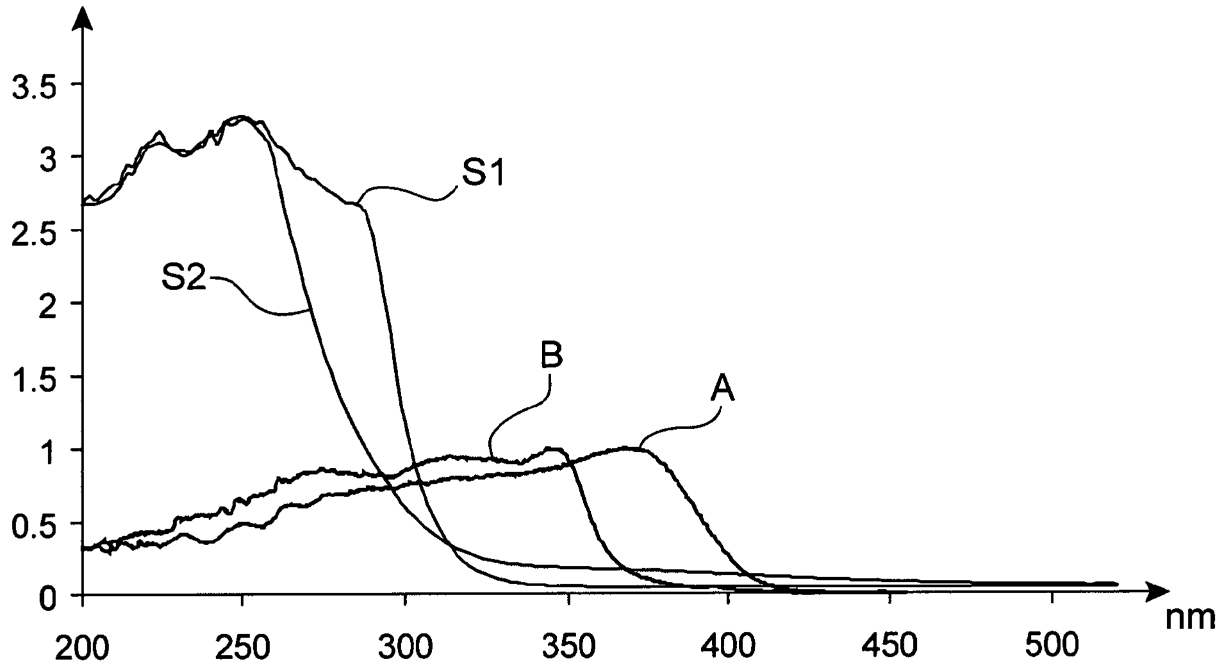


FIG. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **FACCHETTI, A. et al.** *Chem. Comm.,* 2004, 1770-1771
- **LATVA et al.** *Journal of Luminescence,* 1997, vol. 75, 149-169
- **CHATTERTON et al.** Angewandte Chemie. 2005, vol. 44, 7595-7598
- **NONAT et al.** *Chemistry, a European Journal,* 2006, vol. 12, 7133-7150
- **PETOUD et al.** *Journal of the American Chemical Society,* 2003, vol. 125, 13324-13325
- **MOORE et al.** *Inorganic Chemistry,* 2007, vol. 46, 5468-5470
- *ChemPhysChem,* 2001, vol. 2, 769-773
- **CHAUVIN et al.** *Spectroscopy Letters,* 2004, vol. 37, 517-532
- **DE MELLO et al.** *Advanced Materials,* 1997, vol. 9, 230-232
- **MUKKALA et al.** *Helvetica Chimica Acta,* 1992, vol. 75, 1621-1632
- **HOVINEN et al.** *Organic Letters,* 2001, vol. 3, 2473-2476
- **LÓPEZ et al.** *Inorganica Chimica Acta,* 2004, vol. 357, 3525-3531
- **VÖGTLE et al.** *ChemPhysChem,* 2001, vol. 2, 769-773
- **MELLO et al.** *Advanced Materials,* 1997, vol. 9, 230-232